(19) Europäisches Patentamt
European Patent Office
Office européen des brevets

(11) Publication number: **0 475 619 B1**

(12) **EUROPEAN PATENT SPECIFICATION**

(45) Date of publication of patent specification: **11.10.95**

(51) Int. Cl.$^6$: **C07H 13/04**, C07H 13/06, C07H 13/08

(21) Application number: **91307776.4**

(22) Date of filing: **23.08.91**

# (54) Catalyzed sucrose-6-ester process.

(30) Priority: **27.08.90 US 572816**

(43) Date of publication of application:
**18.03.92 Bulletin 92/12**

(45) Publication of the grant of the patent:
**11.10.95 Bulletin 95/41**

(84) Designated Contracting States:
**AT BE CH DE DK ES FR GB IT LI LU NL SE**

(56) References cited:
**EP-A- 0 352 048**

**Patent Abstracts of Japan vol. 11, no. 297 (C-448)(2744) 25 September 1987 & JP-A-62 087 248 (KURARAY Co Ltd) 21 April 1987**

**Patent Abstracts of Japan vol. 12, no. 479 (C-552)(3326) 14 December 1988 & JP-A-63 196 544 (OSAKA YUKI KAGAKU KOGYO K.K.) 15 August 1988**

(73) Proprietor: **McNEIL-PPC, INC.**
**Van Liew Avenue**
**Milltown**
**New Jersey 08850 (US)**

(72) Inventor: **Vernon, Nicholas M.**
**Trewick, Stainton**
**Barnard Castle,**
**Co. Durham, DL12 8RD (GB)**
Inventor: **Wingard, Jr. Robert E.**
**187 Woodside Road**
**Athens GA 30607 (US)**

(74) Representative: **Fisher, Adrian John et al**
**CARPMAELS & RANSFORD**
**43 Bloomsbury Square**
**London WC1A 2RA (GB)**

EP 0 475 619 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the regioselective esterification of sucrose utilizing a distannoxane diester as a catalyst. In an important aspect of the invention, the esterification reaction is accelerated by a tertiary amine.

### Background of the Invention

The sucrose molecule contains three primary hydroxyl groups and five secondary hydroxyl groups. Therefore, when it is desired to prepare derivatives of sucrose involving reaction of the hydroxyl groups, it can be a major synthesis problem to direct the reaction only to the desired hydroxyl groups. For instance, the artificial sweetener 4,1′,6′-trichloro-4,1′,6′-trideoxygalactosucrose ("sucralose") is derived from sucrose by replacing the hydroxyls in the 4, 1′, and 6′ positions with chlorine. (In the process of making the sweetener, the stereo configuration at the 4 position is reversed - hence the compound is a galactosucrose.) This compound and methods for synthesizing it are disclosed in U. S. Patent Nos. 4,343,934, 4,362,869, 4,380,476, and 4,435,440. The direction of the chlorine atoms to only the desired positions is a major synthesis problem, especially since the hydroxyls that are replaced are of differing reactivity (two are primary and one is secondary; the synthesis is further complicated by the fact that the primary hydroxyl in the 6 position is unsubstituted in the final product). A number of different synthesis routes for the sweetener sucralose have been developed in which the reactive 6 position is first blocked, as by an ester group, prior to the chlorination of the hydroxyls in the 4, 1′, and 6′ positions. This invention relates to one method of esterifying the 6 position of sucrose utilizing a distannoxane diester as a catalyst to direct the esterification to the desired 6 position on the sucrose molecule.

The distannoxane-based preparations of sucrose-6-esters were first described in Navia, PROCESS FOR SYNTHESIZING SUCROSE DERIVATIVES BY REGIOSELECTIVE REACTION, United States Patent Application Serial No. 220,641*, filed on July 18, 1988, and assigned to the same assignee as this application. Navia disclosed that a suitable di(hydrocarbyl)tin-based species, such as dibutyltin oxide, dioctyltin oxide, dibutyltin dimethoxide, and the like, can be combined with a hydroxyl-group containing compound such as a monohydric alcohol or a simple phenol to produce a reactive distannoxane intermediate [i.e., a 1,3-di-(hydrocarbyloxy)-1,1,3,3-tetra(hydrocarbyl)distannoxane], which can then be reacted with sucrose to afford a 1,3-di-(6-O-sucrose)-1,1,3,3-tetra(hydrocarbyl)distannoxane.

Navia also described the preparation of sucrose-6-esters by the treatment of these organotin-sucrose adducts with a suitable acylating agent such as acetic or benzoic anhydride in an appropriate solvent or solvent mixture.

The Navia process for the preparation of sucrose-6-esters (S-6-E) consists of three distinct steps, as is shown by the following set of equations which employ for the sake of exemplification dibutyltin oxide hemihydrate (DBTO•$\frac{1}{2}H_2O$) as the di(hydrocarbyl)tin oxide, n-butanol as the monohydric alcohol, acetic anhydride as the acylating agent, and N,N-dimethylformamide (DMF) as the acylation solvent. In the first step DBTO•$\frac{1}{2}H_2O$ is refluxed with a stoichiometric excess of n-butanol in conjunction with the azeotropic removal of the water of condensation to generate 1,3-dibutoxy-1,1,3,3-tetrabutyldistannoxane monohydrate (DBDS•$H_2O$).

* See EP-A-0352048

(1)

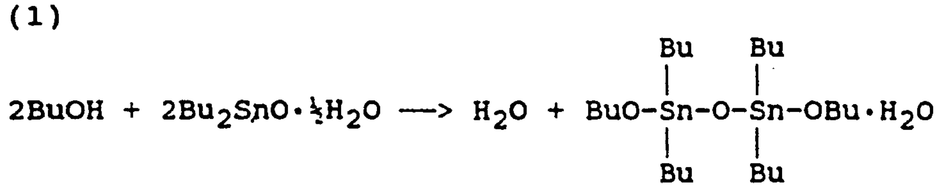

(DBTO•½$H_2O$) (DBDS•$H_2O$)

(2)

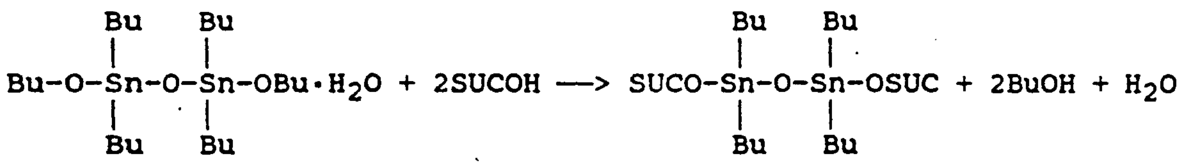

(DBSS)

(3)

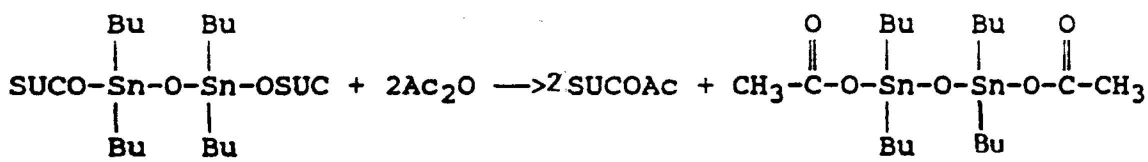

(S-6-A) (DSDA)

In the second step of sucrose-6-acetate (S-6-A) preparation, DBDS•$H_2O$ is reacted with a roughly stoichiometric amount of sucrose (represented as SUCOH) in DMF with removal of water and n-butanol to produce 1,3-di-(6-O-sucrose)-1,1,3,3-tetrabutyldistannoxane, or dibutyldistannoxylsucrose (DBSS). The third step of the process involves treating the hydroxylic solvent-free DBSS solution with a slight stoichiometric excess of acetic anhydride. S-6-A is typically produced in good yields, with only minimal contamination by residual sucrose, sucrose diacetates, and other sucrose monoacetates.

A simpler process for the manufacture of sucrose-6-esters, which is especially suitable for use in the batch-processing mode, was disclosed in Neiditch et al., SUCROSE-6-ESTER PRODUCTION PROCESS, United States Patent Application Serial No. 512,692*, filed on April 23, 1990, and assigned to the same assignee as this application. The Neiditch et al. process involves directly treating sucrose at elevated temperatures with a di(hydrocarbyl)tin oxide (DHTO) in a polar aprotic solvent (such as a tertiary amide) in the presence of a hydrocarbon-like cosolvent, capable of both promoting the dissolution of the DHTO and affecting the codistillative removal of all water generated in the system, to produce an organotin-sucrose adduct. These adducts are distannoxanes of structures identical to those produced by the Navia method [i.e., they are 1,3-di-(6-O-sucrose)-1,1,3,3-tetra(hydrocarbyl)distannoxanes]. The sucrose-substituted distannoxanes produced by the Neiditch et al. process can be readily acylated in situ to afford good yields of S-6-E. This improved two-step process is depicted below for the case of DBTO•½$H_2O$, acetic anhydride, and DMF.

* see European Patent Application EP-A-454,386

(4)

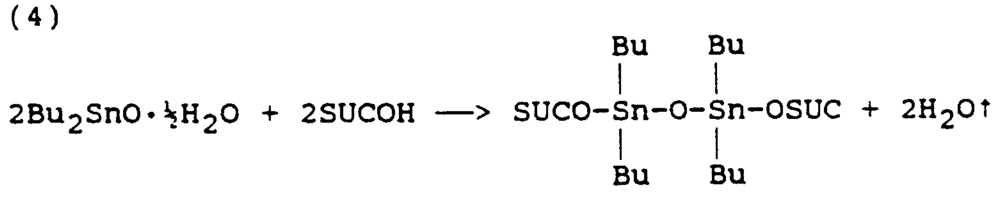

(DBTO·½$H_2O$) (DBSS)

(5)

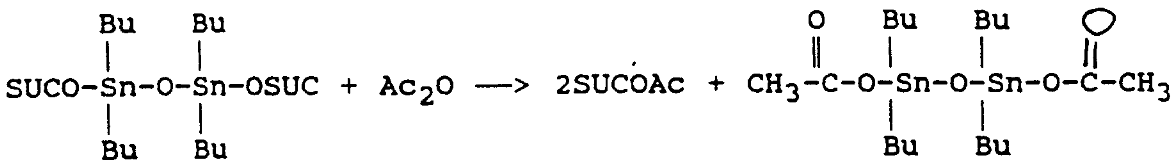

(S-6-A) (DSDA)

Throughout this specification various di(hydrocarbyl)tin-based species, such as dibutyltin oxide and 1,3-dibutoxy-1,1,3,3,-tetrabutyldistannoxane, are shown as possessing water of hydration. These waters of hydration were quantitated by several methods, the most useful of which were Karl Fischer water assays of the substances dissolved in glacial acetic acid. These waters of hydration are released in various of the reactions whose equations are pictured herein, and in all such cases the stoichiometry shown in the equations has been demonstrated in the laboratory.

The economical commercial practice of the Navia and Neiditch et al. processes requires that the organotin end-product, which is a distannoxane diester (DSDE) in both cases, be efficiently recovered and recycled. This issue was addressed in Vernon et al., PROCESS FOR RECOVERY OF ORGANOTIN ESTERS FROM REACTION MIXTURES CONTAINING THE SAME AND RE-USE OF THE RECOVERED ORGANOTIN COMPOUNDS, United States Patent Application Serial No. 512,690*, filed on April 23, 1990, and assigned to the same assignee as this application. Vernon et al. disclose that the anhydrous acylation product mixture of each process, comprised primarily of S-6-E, DSDE, and polar aprotic solvent, can be treated with a relatively small amount of water (molar basis) and the DSDE then almost exclusively extracted as a monohydrate with extraordinary efficiency by an appropriate hydrocarbon-like immiscible solvent. Vernon et al. further disclose that the recovered DSDE•$H_2o$ could be recycled by either: (a) reaction with an alkali metal salt of a hydrocarbonol to regenerate a 1,3-di(hydrocarbyloxy)-1,1,3,3-tetra-(hydrocarbyl)distannoxane for reuse in the process of Navia; or (b) reaction with an alkali metal hydroxide to regenerate a di(hydrocarbyl)tin oxide for reuse in the process of Neiditch et al. These two recycle modes are depicted below, respectively, for the cases of (a) distannoxane diacetate monohydrate (DSDA•$H_2O$), potassium butoxide (KOBu), and DBDS•$H_2O$ (Navia), and (b) DSDA•$H_2O$, sodium hydroxide, and DBTO•½$H_2O$ (Neiditch et al.)

* See European Patent Application EP-A-455,390

(a)

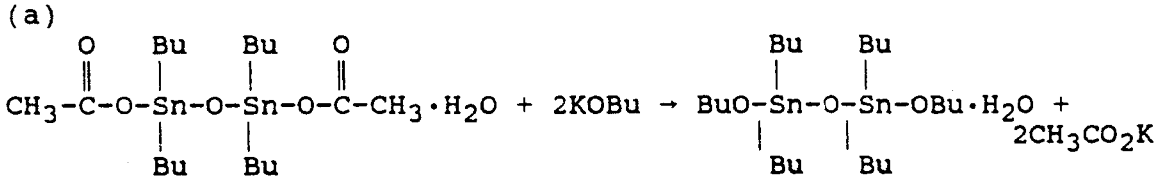

(DSDA•$H_2O$) (DBDS•$H_2O$)

(b)

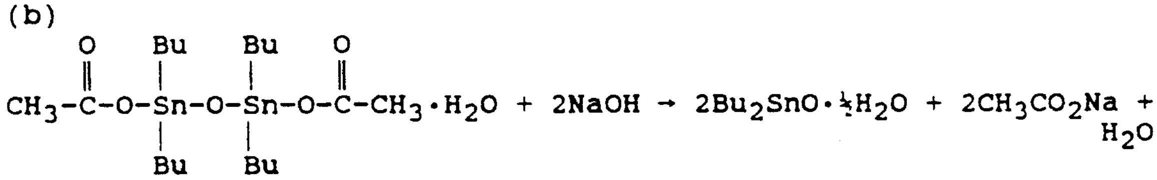

(DSDA•$H_2O$) (DBTO•½$H_2O$)

As may be appreciated by those skilled in the art of industrial chemistry, it would be economically advantageous to be able to recycle recovered DSDE•$H_2O$ without the requirement of regenerating a reactive organotin intermediate, such as a di(hydrocarbyl)tin oxide or a di(hydrocarbyloxy)distannoxane.

Quite surprisingly and unexpectedly, we have discovered that sucrose may be treated with a DSDE•$H_2O$ in a polar aprotic vehicle to (presumably) produce a sucrose-distannoxane diester coordination complex which can be regioselectively acylated in situ, either with or without a tertiary amine accelerator, to produce S-6-E. We have further determined that the DSDE•$H_2O$ may be recovered from acylation media according to the principles set out in Vernon et al., and that it may then be directly reused for S-6-E production following the teachings of this invention.

We have found that the DSDE•$H_2O$ component of this process can be employed in stoichiometric proportions that are substantially less than the corresponding equivalent amount of sucrose undergoing acylation. We have also determined that it is not necessary (although it may be advantageous from the perspective of yield) to remove the water of hydration accompanying the distannoxane derivative prior to treating a reaction mixture of a DSDE•$H_2O$ and sucrose with an acylating agent. We have additionally determined that the benzoylation of sucrose may be catalyzed by DSDA•$H_2O$ without the formation of a detectable amount of sucrose-6-acetate or related acetate esters, thereby inferring the absence of free acetate ions in the reaction mixture.

Furthermore, we have conducted an acetylation reaction, both with and without the use of a tertiary amine accelerator, using sucrose, DSDA-$d_6$ (i.e., distannoxane diacetate prepared from dibutyltin oxide and perdeuterated acetic acid), and unlabelled acetic anhydride. The DSDA-$d_6$ was recovered unchanged from the S-6-A reaction mixture, demonstrating that the distannoxane-to-acetate bond is not broken during the course of the reaction. For these four reasons, and because of the well known sensitivity of 1,3-di-(6-O-sucrose)-1,1,3,3-tetra(hydrocarbyl)distannoxanes to carboxylic acids [instantaneous conversion to sucrose and 1,3-diacyloxy-1,1,3,3-(tetrahydrocarbyl)distannoxanes occurs], we believe that the process of this invention does not involve a 1,3-di-(6-O-sucrose)-substituted distannoxane intermediate of the same nature as those involved in the processes described by Navia and Neiditch et al.

We further believe that the process is catalytic with respect to the DSDE•$H_2O$ (or DSDE) component, and we believe that the reactive intermediate (towards acylation) is a coordinate covalently bonded adduct (i.e., a donor-acceptor or coordination complex) formed between sucrose and a metal atom of the distannoxane diester catalyst. [A coordination complex is defined as a compound containing one or more coordinate covalent bonds, and a coordinate covalent bond is defined as a bond between two atoms in which one of the two atoms supplies both electrons. Tetravalent organotin compounds have a well known propensity for forming penta- and hexacoordinate species if groups with ligand properties, such as hydroxyls, are present. For leading references, see S. David and S. Hanessian, Tetrahedron, 41, 643 (1985) and A. Davies, et al., J. Chem. Soc. Dalton Trans., 297 (1986). For an example of an organotin-mannose derivative containing both five- and six-coordinate tin atoms in the solid state, consult C. Holzapfel, et al., S.

Afr. J. chem., 35, 81 (1982).]

## Brief Summary of the Invention

The process of the invention comprises reacting sucrose with an acylating agent in a polar aprotic reaction vehicle and in the presence of a catalytic quantity of a 1,3-diacyloxy-1,1,3,3-tetra(hydrocarbyl)-distannoxane, or distannoxane diester, for a period of time and at a temperature sufficient to produce a sucrose-6-ester. In a preferred aspect of the invention, the reaction is carried out in the presence of a tertiary amine accelerator.

## The Prior Art

The organotin-mediated regioselective 6-position acylations of sucrose to produce sucrose-6-esters are described in the Navia and the Neiditch et al. patent applications described above. The utility of sucrose-6-esters in a process for producing the artificial sweetener 4,1′,6′-trichloro-4,1′,6′-trideoxygalactosucrose is described, for example, in the Navia and the Neiditch et al. patent applications described above, as well as in Walkup et al., IMPROVED SUCROSE-6-ESTER CHLORINATION, United States Application Serial No. 382,147*, filed July 18, 1989, and assigned to the same assignee as this application.

In a review article entitled REGIOSELECTIVE MANIPULATION OF HYDROXYL GROUPS VIA ORGANOTIN DERIVATIVES, Tetrahedron, Vol. 41, No. 4, pp 643-663 (1985), David et al. disclose the reaction of tin compounds with hydroxyl-group containing compounds to produce stannoxyl compounds, which can then be alkylated or acylated to produce ethers or esters. The reaction of bis(tributyltin) oxide with various carbohydrates (including sucrose), followed by acylation to produce a mixture of esters of varying degrees of substitution, is disclosed. The use of dibutyltin oxide in a reaction with carbohydrates is also disclosed in the article. The authors report the preparation of two dialkylstannylene carbohydrate derivatives, the 2,3-O-dibutylstannylene derivative of methyl 4,6-O-benzylidene-alpha-D-glucopyranoside and 4,6-O-benzylidene-2,3-O-dibutylstannylene-alpha-D-mannopyranoside. The proposed molecular structures of these two stannylene derivatives are shown in Figs. 3 and 4 on page 645 of the article.

Wagner et al., J. Org. Chem., 39, 24 (1974), disclose the preparation of dibutylstannylene derivatives of nucleosides by reacting dibutyltin oxide with nucleosides in refluxing methanol. After stripping off the methanol, the stannylene derivative was acylated by reaction with equimolar quantities of acid chloride and triethylamine.

Holzapfel et al., in "Sucrose Derivatives and the Selective Benzoylation of the Secondary Hydroxyl Groups of 6,1′,6′-Tri-O-tritylsucrose", S. Afr. Tydskr. Chem, 1984,37(3), pages 57-61, disclose the reaction of dibutyltin oxide with 6,1′,6′-tri-O-tritylsucrose, followed by reaction with benzoyl chloride to produce a 72% yield of 3′-O-benzoyl-6,1′,6′-tri-O-tritylsucrose and 9% of the 2-O-benzoate derivative, and minor amounts of the 2,3′-dibenzoate derivative.

1,3-Diisothiocyanato-1,1,3,3-tetrabutyldistannoxane catalyzed transesterification reactions involving substrates such as benzyl alcohol and methyl butyrate are known. For instance, see the following references:

1) J. Otera, S. Ioka, and H. Nozaki, J, Org. Chem., 54, 4013 (1989).

2) For chloro- and hydroxyl-substituted distannoxane transesterification catalysts, see J. Otera, T. Yano, A. Kawabata, and H. Nozaki, Tetrahedron Lett., 2383 (1986).

3) For synthetic applications in the natural products area, see S. Schreiber and H. Meyers, J. Am. Chem. Soc., 110, 5198 (1988); and S. Schreiber, D. Desmaele, and J. Porco, Tetrahedron Lett., 6689 (1988).

## Detailed Description of the Invention

The DSDE-catalyzed process of the invention is shown in the following equations which employ for the sake of exemplification DSDA•$H_2O$ as the 1,3-diacyloxy-1,1,3,3-tetra-(hydrocarbyl)distannoxane monohydrate, acetic anhydride as the acylating agent, and DMF as the acylation solvent. Equation (1) shows how the process is believed to proceed when DSDA•$H_2O$ is employed as catalyst, while Equation (2) shows how the process is believed to proceed when anhydrous DSDA is used. Note that in the following equations the term "DSDA•X" is intended to represent a coordinate covalently bonded adduct or coordination complex formed between DSDA and "X".

* See EP-A-0409549

(1)

$$DSDA \cdot H_2O + SUCOH \rightleftharpoons H_2O + DSDA \cdot SUCOH \xrightarrow{Ac_2O} DSDA \cdot H_2O + S6A + HOAc$$

(2)

$$DSDA \cdot H_2O + SUCOH \longrightarrow H_2O\uparrow + DSDA \cdot SUCOH \xrightarrow[DMF]{Ac_2O} DSDA \cdot DMF + S6A + HOAc$$

The anhydrous amine-accelerated process is detailed in the following set of four sequential equations which for the sake of exemplification employ DSDA•$H_2O$ as the 1,3-diacyloxy-1,1,3,3-tetra(hydrocarbyl)-distannoxane monohydrate, triethylamine as the tertiary amine, acetic anhydride as the acylating agent, and DMF as the acylation solvent.

(3) $DSDA \cdot H_2O + SUCOH \rightarrow H_2O\uparrow + DSDA \cdot SUCOH$

(4) $Ac_2O + Et_3N \rightleftharpoons Et_3N^{+}\text{-}Ac + AcO^{-}$

(5) $DSDA \cdot SUCOH + Et_3N^{+}\text{-}Ac \rightarrow DSDA \cdot DMF + S6A + Et_3NH^{+}$

(6) $DSDA \cdot DMF + SUCOH \rightarrow DSDA \cdot SUCOH + DMF$

Equation (3) shows the reaction of DSDA•$H_2O$ with sucrose to produce a reactive (towards acylation) distannoxane-sucrose coordination complex. Equation (4) shows the reaction of acetic anhydride with triethylamine to produce a highly reactive activated complex (i.e., an acyl trialkylammonium salt), which functions as the actual acylating agent in this process. Equation (5) depicts the reaction of the sucrose coordination complex (produced in equation 3) with the acyl ammonium complex (produced in equation 4) to generate sucrose-6-acetate and spent (i.e., no longer complexed to sucrose) distannoxane diacetate. Equation (6) shows the reaction of free (and as yet unreacted) sucrose with spent DSDA to generate fresh distannoxane-sucrose coordination complex.

The foregoing two sets of equations provide only a highly simplified representation of the interielationships which exist between the various reaction components. These equations are, however, instructive in that they define reaction stoichiometry and demonstrate the catalytic nature of the distannoxane diester component. And, quite importantly, Equations (3) through (6) also show the generation and utilization of the highly reactive activated ammonium acylating complex. It is the presence of this species which provides extraordinarily rapid acylation rates, and which render the amine-accelerated acylation reaction suitable for practice in a continuous processing mode.

The invention may be practiced in any of several different, but closely related, manners. First, with respect to the practice of the invention using the organotin catalyst, but without the amine accelerator, the first method simply involves dissolving sucrose and the requisite amount of DSDE•$H_2O$ catalyst in a polar aprotic solvent (mild heating usually required), and then treating the solution thus produced with a carboxylic acid anhydride at or slightly above room temperature. After the acylation is complete, the solution is treated with a small amount of water and the DSDE•$H_2O$ recovered for reuse by extraction. The acylation product mixture, which at this point consists primarily of S-6-E and lesser amounts of other sucrose monoesters, sucrose diesters, and residual sucrose in a medium consisting of polar aprotic solvent, carboxylic acid, and water, can then be freed of carboxylic acid and dried (e.g., by vacuum distillation) and subjected to chlorination to produce a sucralose-6-ester (TGS-6-E) according to the teachings of the Walkup et al. patent application, cited above.

This mode of practice of the invention is illustrated by Examples 1-12. In Example 5, for instance, 1.00 molar equivalent of sucrose and 0.50 molar equivalent of DSDA•$H_2O$were dissolved in DMF at 75°C, and

the solution thus produced cooled to ambient temperature and treated with 1.10 molar equivalents of acetic anhydride. After stirring for about 18 hr at ambient temperature, the reaction solution was treated with water and extracted with cyclohexane (to recover DSDA•$H_2O$). Partial evaporation of the DMF solution gave a syrup shown by HPLC analysis to contain a 65% yield of S-6-A.

Stoichiometric ratios of tin diester catalyst ranging from 0.10 to 1.00 molar equivalent (basis sucrose) have been demonstrated in the laboratory, producing S-6-E yields ranging from about 35% to about 70%. Laboratory data show that S-6-E yields increase as the stoichiometric amount of catalyst is increased. Both DSDA•$H_2O$ and distannoxane dibenzoate monohydrate (DSDB•$H_2O$) have been shown to be effective catalysts, with DSDA•$H_2O$ demonstrating slightly better performance.

Polar aprotic solvents which have been employed in the laboratory are DMF and N-methyl-2-pyrrolidione (NMP). Other suitable solvents include dimethyl sulfoxide (DMSO), N,N-dimethylacetamide (DMA), and hexamethylphosphoramide (HMPA). When the DSDE•$H_2O$ concentration in the reaction mixture is high, it is advantageous to add a small amount (5-20 vol %) of a hydrocarbon-like cosolvent to keep it in solution. Toluene has demonstrated utility for this purpose. Other useful cosolvents include benzene, mixed xylenes, cyclohexane, methyl tert-butyl ether, chloroform, and the like.

Stoichiometric ratios of carboxylic acid anhydride ranging from about 1.00 to about 4.00 molar equivalents (basis sucrose) have demonstrated experimental utility. Preferred stoichiometric ratios are in the range of from about 1.10 to about 1.80 molar equivalents. Stoichiometric ratios below about 1.10 molar equivalents can lead to an undesirable amount of unreacted sucrose in the final product, while ratios above about 1.80 can cause the formation of undesirable sucrose mono- and diesters.

Acylation reaction temperatures ranging from about 0°C to about 60°C have demonstrated experimental utility. This is not considered to be a particularly critical aspect of the invention, although acylation reaction temperature affects the rate of acylation and excessively high temperatures can increase the production of undesirable sucrose esters. Preferred acylation temperatures range from about 20°C to about 45°C.

Both acetic anhydride and benzoic anhydride have been shown to be effective acylating agents. Acetic anhydride appears to be slightly superior. This is believed to be a result of either steric factors or inherent reactivity, or perhaps some combination of both. A variety of other carboxylic acid anhydrides would be expected to function effectively in the practice of the invention.

DSDE•$H_2O$-catalyzed acylations are generally substantially slower than those involving a 1,3-di-(6-O-sucrose)-substituted distannoxane. This is presumably a result of both the presence of a coordinate covalent bond between the 6-oxygen of sucrose and a tin atom (rather than a normal covalent bond), and the competition between sucrose and other species, such as water, solvent, and the carboxylic acid, for the coordination sites around the tin atom. DSDE•$H_2O$-catalyzed acylations can require from about 2 hr to about 48 hr to reach completion. The rate of acylation is dependent upon a number of variables, which include catalyst stoichiometry (increasing catalyst concentration relative to sucrose increases the rate of acylation), activity of the catalyst (e.g., DSDA•$H_2O$ appears to be a more active catalyst than DSDB•$H_2O$), reactivity of the carboxylic acid anhydride (e.g., acetic anhydride is more reactive than benzoic anhydride), and the reaction temperature and the relative concentration of the reactive species (as the acylation is a multi-order process).

DSDE•$H_2O$ may be recovered for reuse according to the teachings of Vernon et al., cited above. The acylation mixture is treated with a small amount of water and the DSDE•$H_2O$ extracted in an essentially quantitative manner by contacting the mixture with a hydrocarbon such as toluene, cyclohexane, n-heptane, 2,2,4-trimethylpentane, or mixtures thereof, or an ether such as diethyl ether, di(n-propyl) ether, methyl tert-butyl ether, or the like. The volatile extraction solvent is removed by, for example, vacuum evaporation to provide the recovered DSDE•$H_2O$ as a (typically) viscous oil which may then be redissolved in the polar aprotic solvent along with sucrose and the acylation process repeated.

The second mode for the practice of the invention involves the use of a DSDE•$H_2O$ catalyst in a dehydrated or partially dehydrated reaction system, as is illustrated by Examples 13-18. The practice of this aspect of the invention begins by forming a slurry of sucrose and a distannoxane diester monohydrate in a mixed solvent system consisting of a polar aprotic solvent (as above) and a hydrocarbon-like cosolvent capable of removing all or part of the water of hydration of the distannoxane diester (plus any water present from the use of wet reactants or solvents) by codistillation. After removal of the water, the normally biphasic (but solids-free) reaction mixture is treated with a carboxylic acid anhydride at or slightly above room temperature. After the acylation is complete, the mixture is treated with a small amount of water and the DSDE recovered by extraction for reuse. The acylation product mixture may then be further processed (i.e., the water, carboxylic acid, and residual extraction solvent removed) and subjected to chlorination to make TGS-6-E.

In Example 16, for instance, 1.00 molar equivalent of sucrose and 1.05 molar equivalents of DSDA•$H_2O$ were slurried in an 8:3 (by volume) mixture of DMF and cyclohexane, and the mixture vigorously refluxed for 60 min in a reaction vessel equipped with a refluxive water separator. This removed 62% of the original DSDA•$H_2O$ water of hydration. The solids-free reaction mixture was then cooled to ambient temperature and treated with 1.10 molar equivalents of acetic anhydride and stirred for about 18 hr. Following this, the reaction mixture was treated with water, extracted with cyclohexane (to recover DSDA•$H_2O$), and partially evaporated to give a DMF-based syrup shown by HPLC analysis to contain an 82% yield of S-6-A.

Stoichiometric ratios of catalyst ranging from 0.25 to 1.50 molar equivalents (basis sucrose) have been demonstrated in the laboratory, producing S-6-E yields ranging from about 50% to over 80%. Laboratory data shows that S-6-E yields increase as the stoichiometric amount of catalyst is increased. (This is believed to be due to, at least in part, the fact that increased amounts of the DSDE catalyst enhance, presumably as the result of coordination complex formation, the solubility of sucrose in the biphasic reaction media.) Both fully and partially dehydrated DSDA•$H_2O$ and DSDB•$H_2O$ catalysts have proven effective, with other distannoxane diesters also expected to prove useful.

Cosolvents capable of codistillatively removing the water of hydration include saturated hydrocarbons, aromatic hydrocarbons, chlorinated hydrocarbons, ketones, and ethers. A very wide range of solvents appear to be suitable for use as cosolvents in the invention. The primary criteria for a cosolvent are that it produce a mixture with the polar aprotic solvent, the DSDE•$H_2O$, and the sucrose which refluxes with an internal reaction temperature within the range of from about 75°C to about 125°C, that it codistill the water of hydration of the DSDE•$H_2O$, and that it not render key reaction components (e.g., sucrose) insoluble.

Cosolvents which are immiscible with water and which do form a constant-composition minimum-boiling azeotrope with water are preferred, but the cosolvent does not have to be capable of forming a constant-boiling azeotrope of constant composition with water to be an effective cosolvent for the practice of the current invention. Nor is it necessary that the cosolvent be immiscible with water. It is necessary only that the cosolvent be capable of codistilling the water of hydration from the reaction medium.

Preferred cosolvents for reasons of chemical stability, efficiency of water removal, cost, and boiling point include cyclohexane, n-heptane, and isooctane (2,2,4-trimethylpentane). The preferred dehydration temperature is between the range of about 85°C to about 105°C. Temperatures below about 85°C can result in an unnecessarily slow dehydration, while temperature greater than about 105°C can result in decomposition.

Reaction temperatures are typically controlled in an empirical manner by adjusting the ratio of the polar aprotic solvent to the lower boiling cosolvent. Solvent to cosolvent ratios (by volume) of from about one-to-one to about ten-to-one are believed useful for the practice of this invention, with ratios of from about eight-to-five to about eight-to-one being preferred.

Solvent to cosolvent ratios are limited by practical considerations. Too much cosolvent will inhibit sucrose solubility and could produce a mixture with a boiling point too low for reasonable dehydration time. Too little cosolvent can limit the rate at which water can be codistilled from the reaction mixture, and can also result in dehydration temperatures high enough to cause thermal degradation of the carbohydrate species.

A wide range of solids (DSDE•$H_2O$ and sucrose) to solvents (polar aprotic solvent and sucrose) ratios are useful for the practice of the invention. This is not considered to be a particularly critical aspect of the invention, provided that there is sufficient polar aprotic solvent present to insure the partial dissolution of the sucrose, and sufficient cosolvent present to insure water removal and to provide a desirable reaction temperature. Experimentally, solids-to-solvents ratios (wt/vol) of from about one-to-two to about one-to-six have demonstrated utility. The more concentrated systems are preferred for reasons of economics and practicality.

The reflux time required for the full or partial dehydration of mixtures of DSDE•$H_2O$ and sucrose is strictly a function of the efficiency of the removal of water from the system by codistillation. The efficiency of water removal from the reaction system is a function of a number of interactive variables. These variables, which to a large extent can be experimentally controlled, include: (a) the internal reaction temperature; (b) the boiling point of the cosolvent; (c) the water content of the codistillate; (d) the rate of heat input to the system; (e) the efficiency of agitation; and (f) the reactor configuration employed.

Full or partial reaction mixture dehydration times of from about 0.5 hr to about 8.0 hr have demonstrated experimental utility. The reflux period is terminated when the desired amount of water has been codistilled from the system. This determination is usually made by a water analysis of the distillate using the Karl Fischer method. Water removal usually accounts for from about 50% to about 120% of the total present, and appears to be a function of the stoichiometric ratio of sucrose to DSDE•$H_2O$ catalyst.

It becomes more and more difficult to achieve complete dehydration of the reaction mixture as the relative amount of catalyst present increases. For example, 96% of the total amount of water present can be removed by codistillation from a mixture of sucrose and 0.50 molar equivalent of DSDA•$H_2O$ in a DMF-cyclohexane solvent system, but only 62% of the total amount of water present can be removed from an essentially identical system containing 1.05 molar equivalents of DSDA•$H_2O$. (This behavior is probably related to a tin-based coordinaton site requirement for hydroxyl groups. It appears that a ratio of about 2 moles of sucrose per mole of DSDE•$H_2O$ must be attained before full dehydration can be accomplished.)

Partially dehydrated reaction systems, such as the latter example, are difficult to further dehydrate without causing extensive decomposition of the sucrose. By appropriate manipulation of the variables described in the preceding paragraph, total required dehydration times in the 1-2 hr range can typically be experimentally achieved.

After completion of water removal, the normally biphasic (but solids-free) reaction mixtures are cooled to around room temperature and acylated as was described above for the first mode of practice of this invention. Recovery and reuse of the DSDE•$H_2O$ catalyst, and conversion of S-6-E to TGS-6-E may also be readily carried out as described above.

The third method of practice involves utilizing the basic chemistries of Navia and Neiditch et al. in conjunction with a stoichiometric insufficiency (relative to sucrose) of the relevant reactive organotin intermediate, which is 1,3-di(hydrocarbyloxy)-1,1,3,3-tetra(hydrocarbyl)distannoxane for the process of Navia and a di(hydrocarbyl)tin oxide for the process of Neiditch et al. In both cases, according to the teachings of this invention, the reactive (towards acylation) 1,3-di-(6-O-sucrose)-1,1,3,3-tetra(hydrocarbyl)distannoxane intermediates are generated in a substantially anhydrous polar aprotic environment in the presence of free (i.e., not covalently bound to tin) sucrose. Reaction with a slight stoichiometric excess (basis sucrose) of acylating agent results in first the consumption of the covalently bound organotin-sucrose adduct to produce S-6-E and DSDE, followed by the DSDE-catalyzed acylation of the free sucrose present. Note that for the case of the practice of the process of Neiditch et al., the stoichiometric insufficiency of reactive DHTO can be produced by removing (by codistillation) less than the required one mole of water (basis DHTO) from what would otherwise be a stoichiometrically sufficient quantity of DHTO.

This mode of practice of the invention is illustrated by Examples 19-24. In Example 22, for illustration, 1.0 molar equivalent of sucrose was treated with 0.50 molar equivalent of dioctyltin oxide (DOTO.$\frac{1}{2}H_2O$) in a refluxing heptane-DMF mixture for 4 hr. The anhydrous reaction mixture, which at that point contained 0.50 molar equivalent of free sucrose and 0.25 molar equivalent of 1,3-di-(6-O-sucrose)-1,1,3,3-tetraoctyldistannoxane, was then cooled to about 5°C and treated with 1.10 molar equivalents of acetic anhydride to afford, after extraction of 1,3-diacetoxy-1,1,3,3-tetraoctyldistannoxane monohydrate (ODSDA•$H_2O$), an 81% yield of S-6-A.

The methodology of practice of this third mode of the invention follows, with the exception of the previously discussed issues of acylation reaction time and free sucrose solubility, the descriptions set out in Navia and Neiditch et al. An outline of the process of Neiditch et al. is included herein following the Examples.

The following table provides the experimental details and yields for the first 24 Examples, which illustrate that aspect of the invention that employs the DSDE catalyst without the amine accelerator:

DISTANNOXANE DIESTER-CATALYZED SUCROSE-6-ESTER PREPARATIONS

| EXP[1] | AGENT[2] | EQUIV[3] | SOLV[4] | COSOLV[5] | ESTER[6] | TIME[7] | TEMP[8] | $H_2O$[9] | MOLAR % YIELD OR % RECOVERY[10] | | | |
|---|---|---|---|---|---|---|---|---|---|---|---|---|
| | | | | | | | | | S6E[11] | MONO[12] | DI[13] | SUC[14] |
| 1 | DSDA | 0.25 | DMF | --- | A | --- | --- | --- | 55.6 | 5.72 | 17.2 | 18.8 |
| 2 | DSDA | 0.25 | DMF | --- | B | --- | --- | --- | 45.4 | 10.9 | 9.36 | 34.5 |
| 3 | DSDB | 0.25 | DMF | --- | B | --- | --- | --- | 41.3 | 18.9 | 8.11 | 40.4 |
| 4 | DSDB | 0.25 | DMF | --- | A | --- | --- | --- | 54.6 | 3.27 | 16.2 | 18.0 |
| 5 | DSDA | 0.50 | DMF | --- | A | --- | --- | --- | 64.8 | 2.70 | 17.0 | 12.6 |
| 6 | DSDA | 0.50 | DMF | --- | B | --- | --- | --- | 63.0 | 8.52 | 8.52 | 20.0 |
| 7 | DSDA | 0.75 | DMF | --- | A | --- | --- | --- | 69.8 | 2.18 | 17.7 | 10.3 |
| 8 | DSDA | 0.75 | DMF | --- | B | --- | --- | --- | 66.0 | 5.54 | 10.6 | 17.4 |
| 9 | DSDA | 1.00 | DMF | $C_7H_8$ | A | --- | --- | --- | 68.6 | 2.47 | 16.5 | 11.1 |
| 10 | DSDA | 1.00 | DMF | $C_7H_8$ | B | --- | --- | --- | 59.4 | 7.87 | 13.4 | 23.4 |
| 11 | ODSDA | 0.50 | DMF | --- | A | --- | --- | --- | 66.1 | 1.92 | 14.9 | 9.49 |
| 12 | DSDA | 0.50 | NMP | --- | A | --- | --- | --- | 52.2 | 3.55 | 14.2 | 28.9 |
| 13 | DSDA | 0.25 | DMF | $C_6H_{12}$ | A | 2.0 | 89 | 123 | 50.8 | 1.23 | 20.3 | 27.6 |
| 14 | DSDA | 0.50 | DMF | $C_6H_{12}$ | A | 2.0 | 92 | 96 | 66.3 | 0.00 | 22.0 | 11.7 |
| 15 | DSDA | 0.75 | DMF | $C_6H_{12}$ | A | 1.5 | 92 | 80 | 67.6 | 0.00 | 21.3 | 11.1 |
| 16 | DSDA | 1.05 | DMF | $C_6H_{12}$ | A | 1.0 | 95 | 62 | 82.0 | 0.76 | 12.4 | 2.57 |
| 17 | DSDA | 1.05 | DMF | $C_6H_{12}$ | B | 1.0 | 95 | 41 | 73.8 | 5.11 | 6.59 | 14.5 |

| EXP[1] | AGENT[2] | EQUIV[3] | SOLV[4] | COSOLV[5] | ESTER[6] | TIME[7] | TEMP[8] | $H_2O$[9] | MOLAR % YIELD OR % RECOVERY[10] S6E[11] | MONO[12] | DI[13] | SUC[14] |
|---|---|---|---|---|---|---|---|---|---|---|---|---|
| 18 | DSDA | 1.50 | DMF | $C_8H_{18}$ | A | 0.75 | 107 | 59 | 81.1 | 1.31 | 15.3 | 2.21 |
| 19 | DBTO | 0.25 | DMF | $C_7H_{16}$ | B | 6.0 | 98 | 152 | 43.9 | 4.18 | 3.39 | 48.5 |
| 20 | DBTO | 0.50 | DMF | $C_7H_{16}$ | B | 6.0 | 99 | 141 | 84.7 | 0.66 | 5.36 | 10.4 |
| 21 | DBTO | 0.50 | DMF | $C_6H_{12}$ | A | 5.0 | 99 | 146 | 79.4 | 1.27 | 14.3 | 5.00 |
| 22 | DOTO | 0.50 | DMF | $C_7H_{16}$ | A | 4.0 | 99 | 116 | 80.7 | 1.20 | 14.7 | 3.39 |
| 23 | DBTO | 0.50 | DMF | $C_6H_{12}$ | B | --- | --- | --- | 51.3 | 16.3 | 15.2 | 20.5 |
| 24 | DBTO | 1.00 | DMF | $C_6H_{12}$ | B | --- | --- | --- | 73.0 | 4.29 | 18.6 | 3.73 |

[1]Example. [2]Organotin agent employed. DSDA stands for 1,3-diacetoxy-1,1,3,3-tetrabutyldistannoxane. DSDB stands for 1,3-dibenzoyloxy-1,1,3,3-tetrabutyldistannoxane. ODSDA stands for 1,3-diacetoxy-1,1,3,3-tetraoctyldistannoxane. DBTO stands for dibutyltin oxide. DOTO stands for dioctyltin oxide. [3]Equivalents of organotin agent employed basis sucrose. [4]DMF is N,N-dimethylformamide. NMP is N-methyl-2-pyrrolidone. [5]$C_7H_8$ is toluene. $C_6H_{12}$ is cyclohexane. $C_7H_{16}$ is n-heptane. $C_8H_{18}$ is isooctane. [6]Sucrose-6-ester prepared. A is for acetate, and B is for benzoate. [7]Time employed for reaction mixture dehydration in hr. [8]Temperature employed for reaction mixture dehydration in °C. [9]Percent of total $H_2O$ produced during reaction mixture dehydration (Karl Fischer method). [10]Molar percent yield or percent recovery as determined by HPLC analysis of purified syrups. [11]Percent yield of sucrose-6-ester. [12]Percent yield of other sucrose monoesters. [13]Percent yield of sucrose diesters. [14]Percent recovery of unreacted sucrose.

Example 1

PREPARATION OF SUCROSE-6-ACETATE USING 0.25 EQUIVALENT DISTANNOXANE DIACETATE

A 1000-ml, three-neck, round-bottom flask, equipped with mechanical stirrer, thermometer, and 60-ml dropping funnel topped with an argon inlet, was charged with 68.5 g (200 mmol) of sucrose, 30.6 g (50.0 mmol) of DSDA•$H_2O$, and 500 ml of DMF. The suspension was heated at 75°C (internal temperature) for 10 min, and the clear solution thus produced cooled to room temperature and treated dropwise over 15 min with 22.5 g (220 mmol) of acetic anhydride dissolved in 50 ml of DMF. The anhydride addition produced a mild (less than 5°C) exotherm.

The formation of S-6-A ($R_f$ 0.4) and the disappearance of sucrose ($R_f$ 0.2) were followed by silica-gel TLC (15:10:2, $CHCl_3$-$CH_3OH$-$H_2O$, sprayed with 5% ethanolic $H_2SO_4$ and charred). After stirring overnight at room temperature under argon, the reaction mixture was treated with water (50 ml), extracted with cyclohexane (2 x 500 ml) to remove DSDA•$H_2O$, and the DMF evaporated (rotary evaporator, mechanical-pump vacuum, 30 °C water bath) to afford a pale-yellow syrup determined by HPLC analysis to contain 42.7 g (111 mmol, 55.6% yield) of sucrose-6-acetate.

Example 2

PREPARATION OF SUCROSE-6-BENZOATE USING 0.25 EQUIVALENT DISTANNOXANE DIACETATE

The experiment of Example 1 was repeated using 49.8 g (220 mmol) of benzoic anhydride for acylation. The formation of sucrose-6-benzoate (S-6-B, $R_f$ 0.5) was monitored using the same TLC system. After stirring for three days, the reaction was worked-up to give a viscous oil determined by HPLC analysis to contain 40.6 g (90.9 mmol, 45.4% yield) of sucrose-6-benzoate.

Example 3

PREPARATION OF SUCROSE-6-BENZOATE USING 0.25 EQUIVALENT DISTANNOXANE DIBENZOATE

The experiment of Example 2 was repeated using 37.1 g (50.0 mmol) of DSDB•$H_2O$ as catalyst. After stirring at room temperature under argon for three days, the reaction was worked-up to produce a syrup determined by HPLC analysis to contain 36.8 g (82.5 mmol, 41.3% yield) of sucrose-6-benzoate.

Example 4

PREPARATION OF SUCROSE-6-ACETATE USING 0.25 EQUIVALENT DISTANNOXANE DIBENZOATE

The experiment of Example 1 was repeated with 34.2 g (100 mmol) of sucrose, 18.6 g (25.0 mmol) of DSDB•$H_2O$, 250 ml of DMF, and 11.2 g (110 mmol) of acetic anhydride to give a viscous oil determined by HPLC analysis to contain 21.0 g (54.6 mmol, 54.6% yield) of sucrose-6-acetate.

Example 5

PREPARATION OF SUCROSE-6-ACETATE USING 0.50 EQUIVALENT DISTANNOXANE DIACETATE

The experiment of Example 1 was repeated using 61.2 g (100 mmol) of DSDA•$H_2O$. After stirring for 20 hr at room temperature under argon, the reaction was worked-up to give a syrup determined by HPLC analysis to contain 49.8 g (130 mmol, 64.8% yield) of sucrose-6-acetate.

Example 6

PREPARATION OF SUCROSE-6-BENZOATE USING 0.50 EQUIVALENT DISTANNOXANE DIACETATE

The experiment of Example 5 was repeated using 49.8 g (220 mmol) of benzoic anhydride for acylation. After stirring for two days at room temperature under argon, the reaction was worked-up to produce a viscous oil determined by HPLC analysis to contain 56.3 g (126 mmol, 63.0%) of sucrose-6-benzoate.

Example 7

PREPARATION OF SUCROSE-6-ACETATE USING 0.75 EQUIVALENT DISTANNOXANE DIACETATE

The experiment of Example 1 was repeated using 91.8 g (150 mmol) of DSDA•$H_2O$. After stirring overnight at room temperature under argon, the reaction was worked-up to give a syrup determined by HPLC analysis to contain 53.6 g (140 mmol, 69.8% yield) of sucrose-6-acetate.

Example 8

PREPARATION OF SUCROSE-6-BENZOATE USING 0.75 EQUIVALENT DISTANNOXANE DIACETATE

The experiment of Example 7 was repeated using 49.8 g (220 mmol) of benzoic anhydride for acylation. After stirring for two days at room temperature under argon, the reaction was worked-up to give a viscous oil determined by HPLC analysis to contain 58.9 g (132 mmol, 66.0% yield) of sucrose-6-benzoate.

Example 9

PREPARATION OF SUCROSE-6-ACETATE USING 1.00 EQUIVALENT DISTANNOXANE DIACETATE

A 1000-ml, three-neck, round-bottom flask, equipped with mechanical stirrer, thermometer, and 60-ml dropping funnel topped with an argon inlet, was charged with 68.5 g (200 mmol) of sucrose, 122 g (200 mmol) of DSDA•$H_2O$, and 500 ml of DMF. The suspension was heated at 85°C (internal temperature) for 10 min, and the clear solution thus produced cooled to room temperature and treated sequentially with 50 ml of toluene and 22.5 g (220 mmol) of acetic anhydride dissolved in 50 ml of DMF. The anhydride addition raised the reaction temperature from 26°C to 30°C. After stirring 21 hr at room temperature under argon, the solution was worked-up as described in Example 1 to afford a syrup shown by HPLC analysis to contain 52.7 g (137 mmol, 68.6% yield) of sucrose-6-acetate.

Example 10

PREPARATION OF SUCROSE-6-BENZOATE USING 1.00 EQUIVALENT DISTANNOXANE DIACETATE

The experiment of Example 9 was repeated using 49.8 g (220 mmol) of benzoic anhydride for acylation. After stirring for two days at room temperature under argon, the solution was worked-up to produce a syrup shown by HPLC analysis to possess 53.0 g (119 mmol, 59.4% yield) of sucrose-6-benzoate.

Example 11

PREPARATION OF SUCROSE-6-ACETATE USING 0.50 EQUIVALENT 1,3-DIACETOXY-1,1,3,3-TETRAOCTYLDISTANNOXANE

Tetraoctyldistannoxane diacetate monohydrate was prepared by dissolving 37.9 g (100 mmol) of DOTO•$\frac{1}{2}H_2O$ in 400 ml of glacial acetic acid at 80°C (about 15 min required). Rotary evaporation (water-aspirator vacuum, 65°C water bath) afforded the product as a pale-yellow viscous oil. The oil was dissolved in 500 ml of DMF, and the solution partially evaporated (rotary evaporator, mechanical-pump vacuum, 30°C water bath) to remove the residual acetic acid (final volume about 300 ml). The yield was assumed to be quantitative (43.0 g, 50.0 mmol).

The experiment of Example 1 was repeated using the above-described DMF solution of the tetraoctyl derivative, 34.2 g (100 mmol) of sucrose, and 11.2 g (110 mmol) of acetic anhydride. After stirring overnight at room temperature under argon, the reaction mixture was worked-up to give a syrup shown by HPLC assay to contain 25.4 g (66.1 mmol, 66.1% yield) of sucrose-6-acetate.

Example 12

PREPARATION OF SUCROSE-6-ACETATE IN N-METHYL-2-PYRROLIDONE SOLVENT USING 0.50 EQUIVALENT DISTANNOXANE DIACETATE

DSDA•$H_2O$ was prepared by dissolving 51.6 g (200 mmol) of DBTO•$\frac{1}{2}H_2O$ in 400 ml of glacial acetic acid at room temperature (about 5 min required). Rotary evaporation (water-aspirator vacuum, 50°C water bath) provided the product as a colorless viscous oil. The oil was dissolved in 750 ml of NMP, and the solution partially evaporated (rotary evaporator, mechanical-pump vacuum, 50°C water bath) to remove the residual acetic acid (final volume about 500 ml). The yield was assumed to be quantitative (61.2 g, 100 mmol).

The experiment of Example 5 was repeated using the above-described NMP solution. After stirring at room temperature overnight, the reaction mixture was worked-up to afford a syrup shown by HPLC analysis

to possess 40.1 g (104 mmol, 52.2% yield) of sucrose-6-acetate.

Example 13

PREPARATION OF SUCROSE-6-ACETATE USING 0.25 EQUIVALENT DISTANNOXANE DIACETATE WITH DEHYDRATION

A 1000-ml, three-neck, round-bottom flask, equipped with mechanical stirrer, thermometer, and Dean-Stark water separator topped with a reflux condenser, was charged with 68.5 g (200 mmol) of sucrose, 30.6 g (50.0 mmol) of DSDA•$H_2O$, 350 ml of DMF, and 150 ml of cyclohexane. The slurry was heated at reflux (89°C reaction temperature) for 2 hr. The contents of the water separator were removed, dissolved in anhydrous isopropanol, and analyzed for water by the Karl Fischer method (1.11 g, 61.6 mmol).

The slurry was cooled to about 5°C, treated dropwise over about 10 min with 22.5 g (220 mmol) of acetic anhydride, and stirred for an additional 60 min at about 5°C. The anhydride addition produced a mild (less than 5°C) exotherm. After stirring overnight at room temperature under argon, the reaction mixture was treated with water (50 ml), extracted with cyclohexane (2 x 500 ml) to remove DSDA•$H_2O$, and the DMF evaporated (rotary evaporator, mechanical-pump vacuum, 30°C water bath) to afford a dark-brown viscous oil determined by HPLC analysis to contain 39.0 g (102 mmol, 50.8% yield) of sucrose-6-acetate.

Example 14

PREPARATION OF SUCROSE-6-ACETATE USING 0.50 EQUIVALENT DISTANNOXANE DIACETATE WITH DEHYDRATION

The experiment of Example 13 was repeated using 61.2 g (100 mmol) of DSDA•$H_2O$ for catalysis. The dehydration temperature was 92°C (2-hr reflux). Work-up provided a syrup containing 50.9 g (132 mmol, 66.3% yield) of sucrose-6-acetate.

Example 15

PREPARATION OF SUCROSE-6-ACETATE USING 0.75 EQUIVALENT DISTANNOXANE DIACETATE WITH DEHYDRATION

The experiment of Example 13 was repeated using 91.8 g (150 mmol) of DSDA•$H_2O$ as catalyst. The dehydration temperature was 92°C (1.5-hr reflux). Work-up afforded a syrup containing 51.9 g (135 mmol, 67.6% yield) of sucrose-6-acetate.

Example 16

PREPARATION OF SUCROSE-6-ACETATE USING 1.05 EQUIVALENTS DISTANNOXANE DIACETATE WITH DEHYDRATION

A 1000-ml, three-neck, round-bottom flask, equipped with mechanical stirrer, thermometer, and Dean-Stark water separator, topped with a reflux condenser, was charged with 68.5 g (200 mmol) of sucrose, 129 g (210 mmol) of DSDA•$H_2O$,400 ml of DMF, and 150 ml of cyclohexane. The slurry was heated to reflux (95°C reaction temperature), and the resulting solids-free mixture refluxed for 60 min. The contents of the water separator were removed, dissolved in anhydrous isopropanol, and assayed for water by the Karl Fischer method (2.32 g, 129 mmol).

The solids-free mixture was cooled to about 20°C, and treated dropwise over about 3 min with 22.5 g (220 mmol) of acetic anhydride. During the anhydride addition, ice-bath cooling was used as needed to keep the reaction temperature below 25°C. After stirring overnight at room temperature, the reaction mixture was worked-up as described in Example 13 to provide a syrup shown by HPLC analysis to contain 62.9 g (164 mmol, 82.0% yield) of sucrose-6-acetate.

Example 17

PREPARATION OF SUCROSE-6-BENZOATE USING 1.05 EQUIVALENTS DISTANNOXANE DIACETATE WITH DEHYDRATION

The experiment of Example 16 was repeated using 49.8 g (210 mmol) of benzoic anhydride for acylation. The dehydration temperature was 95 °C (60-min reflux). Work-up afforded a syrup containing 65.9 g (148 mmol, 73.8% yield) of sucrose-6-benzoate.

Example 18

PREPARATION OF SUCROSE-6-ACETATE USING 1.50 EQUIVALENTS DISTANNOXANE DIACETATE WITH DEHYDRATION

The experiment of Example 16 was repeated using 34.2 g (100 mmol) of sucrose, 91.8 g (150 mmol) of DSDA•$H_2O$, 400 ml of DMF, 150 ml of isooctane, and 11.2 g (110 mmol) of acetic anhydride. The reaction temperature was 107 °C (45-min reflux). Work-up afforded a syrup containing 31.2 g (81.1 mmol, 81.1% yield) of sucrose-6-acetate.

Example 19

PREPARATION OF SUCROSE-6-BENZOATE USING 0.25 EQUIVALENT DIBUTYLTIN OXIDE WITH DEHYDRATION

A 1000-ml, three-neck, round-bottom flask, equipped with mechanical stirrer, thermometer, and Dean-Stark water separator topped with a reflux condenser, was charged with 68.5 g (200 mmol) of sucrose, 12.9 g (50.0 mmol) of DBTO•$\frac{1}{2}H_2O$, 400 ml of DMF, and 200 ml of n-heptane. The slurry was refluxed for 6 hr (98 °C reaction temperature). The contents of the water separator were removed, dissolved in anhydrous isopropanol, and assayed by the Karl Fischer method of water determination (1.36 g, 75.8 mmol).

The slurry was cooled to about 5 °C, treated dropwise over 10 min with 49.8 g (220 mmol) of benzoic anhydride dissolved in 50 ml of ice-cold DMF, and stirred for an additional 60 min at about 5 °C. After stirring overnight at room temperature under argon, the reaction mixture was filtered to remove undissolved sucrose, treated with water (50 ml), extracted with cyclohexane (2 x 500 ml) to remove DSDB•$H_2O$, and the DMF evaporated (rotary evaporator, mechanical-pump vacuum, 30 °C water bath) to afford a syrup determined by HPLC analysis to contain 39.2 g (87.8 mmol, 43.9% yield) of sucrose-6-benzoate.

Example 20

PREPARATION OF SUCROSE-6-BENZOATE USING 0.50 EQUIVALENT DIBUTYLTIN OXIDE WITH DEHYDRATION

The experiment of Example 19 was repeated using 25.8 g (100 mmol) of DBTO•$\frac{1}{2}H_2O$. The reaction temperature was 99 °C (6-hr reflux). In contrast to the directly preceding experiment, this reaction mixture was solids-free throughout the reflux and benzoylation periods. Work-up afforded a syrup containing 75.5 g (169 mmol, 84.7% yield) of sucrose-6-benzoate.

Example 21

PREPARATION OF SUCROSE-6-ACETATE USING 0.50 EQUIVALENT DIBUTYLTIN OXIDE WITH DEHYDRATION

The experiment of Example 19 was repeated using 68.5 g (200 mmol) of sucrose, 25.8 g (100 mmol) of DBTO•$\frac{1}{2}H_2O$, 400 ml of DMF, 100 ml of cyclohexane, and 22.5 g (220 mmol) of acetic anhydride. The dehydration temperature was 99 °C. The reaction was solids-free throughout the reflux and acetylation. Work-up afforded a syrup shown by HPLC analysis to contain 61.0 g (159 mmol, 79.4% yield) of sucrose-6-acetate.

Example 22

PREPARATION OF SUCROSE-6-ACETATE USING 0.50 EQUIVALENT DIOCTYLTIN OXIDE WITH DEHYDRATION

The experiment of Example 19 was repeated using 37.9 g (100 mmol) of DOTO•$\frac{1}{2}H_2O$ and 22.5 g (220 mmol) of acetic anhydride. The dehydration temperature was 99°C (4-hr reflux). This reaction remained solids-free throughout the reflux and acetylation. Work-up afforded a syrup shown by HPLC analysis to contain 62.0 g (161 mmol, 80.7% yield) of sucrose-6-acetate.

Example 23

PREPARATION OF SUCROSE-6-BENZOATE USING 0.50 EQUIVALENT DIBUTYLTIN OXIDE WITHOUT DEHYDRATION

A 500-ml, one-neck, round-bottom flask, equipped with magnetic stir bar and argon inlet, was charged with 17.1 g (50.0 mmol) of sucrose, 6.45 g (25.0 mmol) of DBTO•$\frac{1}{2}H_2O$, 18.1 g (80.1 mmol) of benzoic anhydride, 200 ml of DMF, and 100 ml of cyclohexane. This mixture was stirred at room temperature for 9 days, treated with water (25 ml), and extracted with cyclohexane (2 x 150 ml) to remove DSDB•$H_2O$.Evaporation (rotary evaporator, mechanical pump, 30°C water bath) afforded a syrup shown by HPLC analysis to contain 11.4 g (25.7 mmol, 51.3% yield) of sucrose-6-benzoate.

Example 24

PREPARATION OF SUCROSE-6-BENZOATE USING 1.00 EQUIVALENT DIBUTYLTIN OXIDE WITHOUT DEHYDRATION

The experiment of Example 23 was repeated with 12.9 g (50.0 mmol) of DBTO•$\frac{1}{2}H_2O$. After stirring for 9 days, work-up provided a syrup which contained 16.3 g (36.5 mmol, 73.0% yield) of sucrose-6-benzoate.

Example 25

PREPARATION OF SUCROSE-6-ACETATE USING 1.00 EQUIVALENT OF RECYCLED DISTANNOXANE DIACETATE WITH DEHYDRATION

The cyclohexane extracts produced in Example 16 were combined, washed with 200 ml of 50% saturated aqueous brine, and subjected to rotary evaporation (water-aspirator vacuum, 30°C water bath followed by mechanical-pump vacuum, 40°C water bath) to produce 122.6 g (200 mmol) of recovered DSDA•$H_2O$. This viscous tan oil was treated with 150 ml of cyclohexane, and the resulting solution employed in a repeat of Example 16. The reaction temperature was 93°C (60-min reflux). Work-up afforded a syrup containing 60.5 g (158 mmol, 78.8% yield) of sucrose-6-acetate.

Example 26

GENERIC PROCEDURE FOR THE PREPARATION OF DISTANNOXANE DIESTERS

DBTO•$\frac{1}{2}H_2O$ (103 g, 0.40 mol) iss refluxed with acetic or benzoic acid (24.1 g or 49.1 g, 0.40 mol) in toluene or cyclohexane (200-400 ml) for about 2 hr with the water of reaction being separated in a Dean-Stark trap. The DSDE•$H_2O$ could be used in solution, or crystallized by solvent removal and dissolution in either 200 ml of 5% aqueous acetonitrile (DSDB•$H_2O$) or 100 ml of 5% aqueous DMF (DSDA•$H_2O$). DSDA•$H_2O$ displays a melting point of 57-8°C[a], and provids a satisfactory elemental analysis (calcd for $C_{20}H_{42}O_5Sn_2$•$H_2O$:C, 39.39; H, 6.83. found: C, 38.87; H, 6.83). DSDB•$H_2O$ displays a melting point of 94-6°C, and also provids a satisfactory elemental analysis (calcd for $C_{30}H_{46}O_5Sn_2$•$H_2O$:C, 48.55; H, 6.52. found: C, 47.26; H, 6.24).

When a tertiary amine is employed to accelerate the acylation reaction, the invention may be carried out in a variety of different modes. These modes are differentiated, in part, by: (a) the extent of dehydration

[a]lit mp 58-60°C [D. Alletson, et al., J. Chem. Soc., 5469, (1963)].

of the DSDE•$H_2O$ employed as organotin agent; (b) the nucleophilicity of the tertiary amine employed; (c) the method of recovery employed for the S-6-E product; (d) the method of recovery and recycle employed for the tertiary amine component; and (e) the method of recovery and recycle used for the DSDE•$H_2O$ component. Those methods of practice which involve continuous processing are preferred.

Five modes which use the tertiary amine accelerator are discussed below in some detail. These five modes of practice are representative, and a number of other modes of practice of the invention may be developed by the modification of various aspects of the disclosed modes. The first two of these modes involve batchwise processing, while the latter three involve continuous processing. The first mode simply involves dissolving sucrose and the requisite amount of DSDE•$H_2O$ catalyst in a polar aprotic solvent (mild heating usually required) and then sequentially treating the solution thus produced with a low-boiling (relative to the polar aprotic solvent) tertiary amine and a carboxylic acid anhydride at or slightly below room temperature. After the acylation is complete, the solution is treated with a small amount of water and the DSDE•$H_2O$ recovered for reuse by extraction. The acylation product mixture, which at this point consists primarily of S-6-E (and lesser amounts of other sucrose esters and residual sucrose) in a medium consisting of polar aprotic solvent, water, tertiary amine, and carboxylic acid, can then be freed of low-boiling tertiary amine (e.g., by vacuum evaporation or treatment with a cation exchange resin), carboxylic acid (e.g., by vacuum evaporation or treatment with an anion exchange resin), and water (e.g., by vacuum evaporation or treatment with a dehydrating agent) to produce a syrup from which the S-6-E may be isolated by crystallization or precipitation techniques. Alternatively, purified and dried DMF-based syrups may be subjected to chlorination to produce a sucralose-6-ester according to the teachings of Walkup et al., cited above.

This mode of practice of the invention is illustrated by Examples 27 and 28 herein. In Example 27, by way of illustration, 1.00 molar equivalent of sucrose and 0.50 molar equivalent of DSDA•$H_2O$ were dissolved in DMF at 75°C, and the solution thus produced cooled to room temperature and treated sequentially with toluene, triethylamine (1.10 molar equivalents), and acetic anhydride (1.10 molar equivalents). After stirring for 30 min at ambient temperature, the reaction solution was treated with water and extracted with cyclohexane (to recover DSDA•$H_2O$). Vacuum evaporation was employed to remove triethylamine, acetic acid, water, and a portion of the DMF to give a syrup found to contain a 64% yield of S-6-A.

Stoichiometric ratios of DSDE catalyst, nature and proportion of the acylating agent, and the nature and proportion of polar aprotic reaction vehicle that were discussed above (in connection with the aspect of the invention which uses no tertiary amine accelerator) are also applicable to the amine-accelerated aspect of the invention. Both DSDA•$H_2O$ and DSDB•$H_2O$ have been shown to be effective catalysts, with DSDB•$H_2O$ demonstrating slightly better performance in the amine-accelerated aspect of the invention. This performance difference may be result of a decreased tendency of the more sterically hindered DSDB•$H_2O$ to become involved in side reactions caused by the tertiary amine.

When the DSDE•$H_2O$ concentration in the reaction mixture is high, it is advantageous to add a small amount (5-20 vol %) of a hydrocarbon-like cosolvent to keep it in solution. Toluene has demonstrated utility for this purpose. Other useful cosolvents include benzene, mixed xylenes, cyclohexane, methyl tert-butyl ether, chloroform, and the like.

Stoichiometric ratios of low-boiling tertiary amine ranging from about 1.00 to about 1.25 molar equivalents (basis sucrose) have been employed. Preferred stoichiometric ratios are in the range of from about 1.05 to about 1.10 molar equivalents, and are equal to the molar equivalents of carboxylic acid anhydride being employed in that specific process. The use of less tertiary amine than acid anhydride (molar basis) will cause a significant decrease in the rate of acylation. The use of a stoichiometric excess of tertiary amine can result in lowered yields if side reaction with the organotin catalyst, initiated by the tertiary amine, occur.

Acylation reaction temperatures similar to those discussed above with respect to the DSDE-catalyzed acylation without the use of amine accelerator can be employed.

Both acetic anhydride and benzoic anhydride have been shown to be effective acylating agents. Benzoic anhydride appears to provide superior yields in the amine-accelerated aspect of the invention. This is believed to be because the lower inherent reactivity of benzoic anhydride results in the occurrence of fewer side reactions. A wide variety of other carboxylic acid anhydrides can be employed in the invention.

A large assortment of low-boiling tertiary amines are suitable for use in this mode of practice of the invention. Key criteria for this process component are that it not produce an excessive amount of side reaction with the DSDE•$H_2O$ catalyst (some tertiary amines, such as for example imidazole and 1,8-diazabicyclo[5.4.0]undec-7-ene, fairly rapidly impair the utility of these catalysts), that it possess sufficient nucleophilicity to react with the carboxylic acid anhydride to produce an acyl ammonium activated complex,

and that it be easily removed (by, for example, vacuum evaporation) to provide for the ready isolation of the S-6-E as either a solid or purified syrup. Tertiary amines which have been employed successfully include trialkylamines such as trimethylamine (TMA), triethylamine (TEA), and diisopropylethylamine (DEA), and aromatic heterocyclic amines such as pyridine and 2,6-lutidine (2,6-dimethylpyridine). Other appropriate amines would include diethylmethylamine, dimethylethylamine, and the picolines (methylpyridines). Preferred tertiary amines include TMA and TEA because of cost and ease of removal by vacuum evaporation.

These amine-accelerated acylations are generally substantially faster than those involving 1,3-di-(6-O-sucrose)-substituted distannoxanes. This is presumably a result of the great reactivity of the acyl ammonium salt, which is the de facto acylating agent. Amine-accelerated acylations can require from about 3 min to about 60 min to reach completion. The rate of regioselective acylation is dependent upon a number of variables, which include catalyst stoichiometry (increasing catalyst concentration relative to sucrose increases the rate), activity of the catalyst (e.g., DSDA•$H_2O$ appears to be a more active catalyst than DSDB•$H_2O$), nucleophilicity of the tertiary amine employed (e.g., triethylamine is more nucleophilic than pyridine and hence produces a higher concentration of the acyl ammonium salt thereby affording a faster reaction rate), reactivity of the carboxylic acid anhydride (e.g., acetic anhydride is more reactive than benzoic anhydride), and the reaction temperature and the relative concentration of the reactive species (as the acylation is a multi-order process).

DSDE•$H_2O$ may be recovered for reuse according to the teachings of Vernon et al., as discussed above.

The low-boiling tertiary amine may be recovered by fractional distillation techniques following the extraction of the DSDE•$H_2O$. For example, a DSDE-free DMF-based S-6-E solution can be "stripped" (using, for example, a thin-film evaporator) of low-boiling tertiary amine (such as trimethylamine, bp 3°C), water (bp 100°C), and a portion of the DMF (bp 153°C) to produce a distillate from which the tertiary amine can be readily recovered by fractional distillation. Alternatively, the DSDE-free DMF-based S-6-E solution can be carefully fractionally distilled to recover the tertiary amine. In those cases where the carboxylic acid present interferes with tertiary amine recovery, the acid can be removed by the use of an appropriate anion exchange resin following the DSDE•$H_2O$ extraction. Likewise, for those cases where the water present interferes with the recovery of anhydrous tertiary amine, the.water can be removed by the use of a dehydrating agent (such as molecular sieves) following the DSDE•$H_2O$ extraction.

The second mode for the practice of the amine-accelerated aspect of the invention involves the use of a DSDE•$H_2O$ catalyst in a dehydrated or partially dehydrated reaction system as is illustrated by Examples 29 through 45 herein. This mode of practice is conducted in a manner similar to that described above for that mode of practice of the invention conducted under dehydrated or partially dehydrated reaction conditions, but containing no amine accelerator. After the acylation is complete, the solution is treated with a small amount of water and the DSDE•$H_2O$ recovered by extraction for reuse. The acylation product mixture may then be further processed (i.e., the water, tertiary amine, carboxylic acid, and residual extraction solvent removed) and the S-6-E isolated as a solid (e.g., by crystallization or precipitation) or, for the case of DMF-based syrups, subjected to chlorination to make a sucralose-6-ester.

In Example 36, for illustration, 1.00 molar equivalent of sucrose and 1.00 molar equivalent of DSDB•$H_2O$ were slurried in a 4:1 (by volume) mixture of DMF and cyclohexane, and the mixture vigorously refluxed for 30 min in a reaction vessel equipped with a refluxive water separator. This removed 40% of the original DSDB•$H_2O$ water of hydration. The solids-free reaction mixture was then cooled to ambient temperature and treated sequentially with 1.10 molar equivalents of triethylamine and 1.10 molar equivalents of acetic anhydride. After stirring for about 30 min, the reaction mixture was treated with water, extracted with cyclohexane (to recovery DSDB•$H_2O$), and partially evaporated (to remove triethylamine, water, acetic acid, and a portion of the DMF) to produce a syrup shown by HPLC analysis to contain an 84% yield of sucrose-6-acetate.

Stoichiometric ratios of DSDE catalyst, nature and proportion of cosolvents, reaction temperatures, reaction time for the dehydration step, and nature and proportion of the acylating agent, are as discussed above for the second mode of practice for the nonamine accelerated aspect of the invention.

After completion of water removal, the normally biphasic (but solids-free) reaction mixtures are cooled to room temperature or below and treated with low-boiling tertiary amine and then acylated as was described above for the first mode of the amine-accelerated aspect of the invention. Recovery and reuse of both the DSDE•$H_2O$ catalyst and low-boiling tertiary amine, isolation of the S-6-E in either solid or purified syrup form, and conversion of S-6-E to TGS-6-E may also be carried out as described above. The process of the second mode follows, in general, the criteria for tertiary amine and anhydride stoichiometry, polar aprotic solvent structure, and acylation temperature set out in the discussion of the first mode.

The third mode of practice is diagrammed in Scheme I, shown as Fig. 1. This is a continuous processing mode which involves the use of a polar aprotic acylation solvent, a hydrocarbon-like cosolvent for both the recycle extraction and dehydration of the DSDE•$H_2O$ organotin agent, a nonvolatile (i.e., substantially higher boiling than the polar aprotic solvent) tertiary amine to generate the activated complex for acylation, the isolation of solid S-6-E by crystallization (or precipitation), the recovery and recycle (in a single process stream) of both the DSDE•$H_2O$ catalyst and the nonvolatile amine, and the removal of the carboxylic acid byproduct from the process stream by extraction with a precise stoichiometric amount of aqueous sodium hydroxide. The solid S-6-E produced by this process is suitable for chlorination to sucralose-6-ester.

This mode of practice of the invention is demonstrated by attached Example 46, which details three repetitive cycles of a process which employs DMF as the polar aprotic solvent, toluene as the extraction-dehydration cosolvent, DSDB•$H_2O$ as the organotin catalyst, N,N-dimethyloctylamine (DMOA, bp 195°C) as the nonvolatile tertiary amine, benzoic anhydride as the acylating agent, and the crystallization of S-6-B from acetone. The process began by dissolving sucrose (1.00 molar equivalent) and DSDB•$H_2O$ (0.60 molar equivalent) in DMF at about 80°C. This solution was dehydrated by codistillation with toluene under reduced pressure at about 90°C. The mixture was then cooled to about 20°C and sequentially treated with DMOA (1.10 molar equivalents) and benzoic anhydride (1.10 molar equivalents) with continued cooling to maintain an approximately 20°C reaction temperature.

After stirring briefly, the DMF was removed (for direct recycle) by vacuum evaporation to produce a viscous oil (containing DSDB, S-6-B, benzoic acid, and DMOA) which was treated with acetone to give a solution from which S-6-B crystallized. The carbohydrate was isolated by filtration and vacuum dried. The filtrate was vacuum evaporated to remove the acetone (for recycle) producing a viscous oil (containing DSDB, benzoic acid, and DMOA) which was contacted with toluene and water containing 1.10 molar equivalents of sodium hydroxide. The benzoic acid was quantitatively extracted into the aqueous phase as its sodium salt, while the DSDB•$H_2O$ and DMOA remained in the hydrocarbon phase. (In the attached Example the aqueous sodium benzoate solution was discarded. In a commercial operation this salt would be recovered and reconverted to benzoic anhydride.)

The toluene was then removed (for recycle) by vacuum evaporation to produce a relatively anhydrous viscous oil composed of DSDB and DMOA, which were quantitated by, respectively, atomic absorption spectrophotometry and gas chromatography. A toluene solution of the assayed oil was then treated with sucrose, DMF, and make-up DSDB•$H_2O$ and the process repeated, with make-up DMOA added at the appropriate part of the procedure. Example 46 shows three consecutive cycles of this process with isolated S-6-B yields averaging 71.5% with an average purity (by HPLC analysis) of 92.7%. DSDB•$H_2O$ recovery averaged 96.7%, and DMOA recovery averaged 90.9%.

The process of Scheme I follows, in general, the criteria for organotin agent, tertiary amine, and anhydride stoichiometry, catalyst and polar aprotic solvent structure, and acylation temperature set out in the discussion of the first mode of practice of the amine-accelerated aspect of the invention. The process of Scheme I also follows, in general, the criteria regarding the use of dehydration cosolvents set out in the discussion of the second mode of practice of the amine-accelerated aspect of the invention. Note that it would be possible to practice the process of Scheme I using anhydrides other than benzoic anhydride, provided that a suitable S-6-E crystallization solvent is utilized.

DMOA, 2,4,6-collidine (2,4,6-trimethylpyridine, bp 171°C), and N,N-dimethyldodecylamine (DMDA, bp 110°C at 3 mm of Hg) have proven effective for the practice of the process of Scheme I. A variety of other high-boiling tertiary amines, such as tri-n-butylamine (bp 216°C), tri-n-octylamine (bp 365°C), and N-methyldi-n-octylamine (bp 162°C at 15 mm of Hg), can also be used as the nonvolatile tertiary amine component of this process.

The fourth mode of practice of the invention is pictured in Scheme II, shown as Fig. 2. This is a continuous processing mode which involves the use of a polar aprotic acylation solvent, a hydrocarbon-like cosolvent for both the recycle extraction and dehydration of the DSDE•$H_2O$ organotin agent, a volatile (i.e., substantially lower boiling than the polar aprotic solvent) tertiary amine to generate the activated complex for acylation, the isolation of solid S-6-E by crystallization (or precipitation), the recovery and recycle of the DSDE•$H_2O$ catalyst, and the removal of the carboxylic acid byproduct from the process stream by extraction with a precise stoichiometric amount of aqueous sodium hydroxide. The solid S-6-E generated by this process is suitable for chlorination to produce sucralose-6-ester.

This mode of practice of the invention is demonstrated by attached Example 47, which describes five repetitive cycles of a process which employees DMF as the polar aprotic solvent, toluene as the extraction-dehydration cosolvent, DSDB•$H_2O$ as the organotin agent, triethylamine as the volatile tertiary amine, benzoic anhydride as the acylating agent, and the crystallization of S-6-B from acetone. The process was

started by dissolving sucrose (1.00 molar equivalent) and DSDB•$H_2O$ (0.60 molar equivalent) in DMF at about 80°C. This solution was dehydrated by codistillation with toluene at about 90°C under reduced pressure. The mixture was then cooled to about 20°C and treated sequentially with TEA (1.10 molar equivalents) and benzoic anhydride (1.10 molar equivalents) with continued cooling to maintain an approximately 20°C reaction temperature.

After stirring briefly, the volatile amine and DMF were removed (see the earlier discussion regarding the separation and recycle of these two reaction components) by vacuum evaporation to produce a viscous oil (containing DSDB, S-6-B, and benzoic acid) which was treated with acetone to give a solution from which S-6-B crystallized. The carbohydrate was isolated by filtration and vacuum dried. The filtrate was vacuum evaporated to remove the acetone (for recycle) producing a viscous oil (containing DSDB and benzoic acid) which was contacted with toluene and water containing 1.10 molar equivalents of sodium hydroxide. The benzoic acid was quantitatively extracted into the aqueous phase as its sodium salt, while the DSDB•$H_2O$ remained in the hydrocarbon phase. (In the attached Example the aqueous sodium benzoate solution was discarded. In a commercial operation this salt would be recovered and reconverted to benzoic anhydride.)

The toluene was then removed (for recycle) by vacuum evaporation to afford a relatively anhydrous viscous oil composed of DSDB (quantitated by atomic absorption spectrophotometry). A toluene solution of the assayed oil was then treated with sucrose, DMF, and make-up DSDB•$H_2O$ and the process repeated. Example 47 shows five consecutive cycles of this process with isolated S-6-B yields averaging 71.9% with an average purity (by HPLC analysis) of 92.4%. DSDB•$H_2O$ recovery averaged 97.1%.

The process of Scheme II follows, in general, the criteria for organotin catalyst, tertiary amine, and anhydride stoichiometry, organotin catalyst, tertiary amine, and polar aprotic solvent structure, and acylation temperature set out in the discussion of the first mode of practice of of the amine-accelerated aspect of the invention. The process of Scheme II also follows, in general, the criteria regarding the use of dehydration cosolvents set out in the discussion of the second mode of practice of the amine-accelerated aspect of the invention. Note that it would be possible to practice the process of Scheme II using anhydrides other than benzoic anhydride, provided that a suitable S-6-E crystallization solvent is utilized.

The fifth mode is depicted in Scheme III, shown as Fig. 3. This is a continuous processing mode which involves the use of a polar aprotic acylation solvent, a hydrocarbon-like cosolvent for both the recycle extraction and dehydration of the DSDE•$H_2O$ organotin agent, a volatile (i.e., substantially lower boiling than the polar aprotic solvent) tertiary amine to generate the activated complex for acylation, the isolation of the S-6-E as a purified syrup, the recovery and recycle of the DSDE•$H_2O$ component, and the removal of both the volatile tertiary amine and the carboxylic acid byproduct from the process stream by distillation techniques. The S-6-E syrups generated by this process are suitable for conversion to sucralose-6-ester by chlorination.

This mode of practice of the invention is demonstrated by Example 48, which describes five repetitive cycles of a process which employees DMF as the polar aprotic solvent, cyclohexane as the extraction-dehydration cosolvent, DSDA•$H_2O$ as the organotin agent, triethylamine as the volatile amine, and acetic anhydride as the acylating agent. The process was begun by dissolving sucrose (1.00 molar equivalent) and DSDA•$H_2O$ (1.00 molar equivalent) in DMF at about 80°C. This solution was treated with cyclohexane and dehydrated by codistillation at about 90°C. The mixture was cooled to about 20°C and treated sequentially with TEA (1.10 molar equivalents) and acetic anhydride (1.10 molar equivalents) with continued cooling to maintain an approximately 20°C reaction temperature.

After stirring briefly, the reaction mixture was treated with water and the DSDA•$H_2O$ recovered by extraction with cyclohexane. The DMF-based solution (containing S-6-A, acetic acid, water, and TEA) was then evaporated under reduced pressure to remove the volatile amine, water, acetic acid, and a portion of the DMF (see the earlier discussion regarding the separation and recycle of these reaction components) to produce a syrup which was assayed for S-6-A and DMF content by, respectively, HPLC and gas chromatography. The cyclohexane was then removed (for recycle) from the combined extracts to afford a relatively anhydrous viscous oil composed of DSDA (assayed by atomic absorption spectrophotometry). A cyclohexane solution of the assayed oil was then treated with sucrose, DMF, and make-up DSDA•$H_2O$ and the process repeated. Example 22 shows five consecutive cycles of this process with syrup S-6-A yields averaging 79.0%. DSDA•$H_2O$ recovery averaged 98.8%.

The process of Scheme III follows, in general, the criteria for organotin agent, tertiary amine, and anhydride stoichiometry, organotin agent, tertiary amine, and polar aprotic solvent structure, and acylation temperature set out in the discussion above. The process of Scheme III follows, in general, the criteria regarding the use of dehydration cosolvents set out in the discussion of the second mode of practice of the amine-accelerated aspect of the invention. Additionally, the process of Scheme III follows, in general, the criteria regarding DSDE.$H_2O$ extraction and recycle set out in Vernon et al., discussed above. Note that it is

possible to practice the process of Scheme III using anhydrides which produce a nonvolatile carboxylic acid byproduct, provided that a suitable method (such as extraction followed by neutralization or the use of an anion exchange resin) is utilized to remove the byproduct from the process stream.

The following table provides the experimental details and yields for Examples 27-45 herein.

AMINE-ACCELERATED SUCROSE-6-ESTER PREPARATIONS

| EXP[1] | AGENT[2] | EQUIV[3] | SOLV[4] | COSOLV[5] | ESTER[6] | TIME[7] | $H_2O$[8] | AMINE[9] | MOLAR % YIELD OR % RECOVERY[10] S6E[11] | MONO[12] | DI[13] | SUC[14] |
|---|---|---|---|---|---|---|---|---|---|---|---|---|
| 27 | DSDA | 0.50 | DMF | $C_7H_8$ | A | --- | --- | TEA | 64.0 | 5.09 | 20.3 | 10.6 |
| 28 | DSDB | 0.50 | DMF | $C_7H_8$ | B | --- | --- | TEA | 72.5 | 11.8 | 3.48 | 12.2 |
| 29 | DSDA | 0.50 | DMF | $C_6H_{12}$ | A | 45 | 72 | TEA | 75.1 | 2.85 | 16.7 | 5.34 |
| 30 | DSDA | 0.25 | DMF | $C_6H_{12}$ | A | 60 | 116 | TEA | 62.0 | 5.63 | 18.4 | 14.0 |
| 31 | DSDA | 0.50 | DMF | $C_6H_{12}$ | A | 45 | 66 | PYR | 60.9 | 1.72 | 19.3 | 18.0 |
| 32 | DSDA | 0.50 | DMF | $C_6H_{12}$ | A | 45 | 76 | DEA | 72.6 | 3.24 | 18.3 | 5.91 |
| 33 | DSDB | 0.50 | DMF | $C_6H_{12}$ | A | 30 | 60 | TEA | 76.2 | 2.92 | 15.8 | 5.09 |
| 34 | DSDA | 0.50 | DMF | $C_6H_{12}$ | A | 45 | 78 | LUT | 72.9 | 1.63 | 17.9 | 7.63 |
| 35 | DSDA | 0.50 | DMF | $C_6H_{12}$ | B | 45 | 57 | TEA | 81.6 | 3.09 | 5.66 | 9.65 |
| 36 | DSDB | 1.00 | DMF | $C_6H_{12}$ | A | 30 | 40 | TEA | 83.5 | 1.00 | 12.9 | 2.61 |
| 37 | DSDB | 1.00 | DMF | $C_6H_{12}$ | B | 45 | 46 | TEA | 86.2 | 1.72 | 11.1 | 1.00 |
| 38 | DSDB | 0.75 | DMF | $C_6H_{12}$ | A | 30 | 47 | TEA | 83.0 | 1.30 | 13.9 | 1.80 |
| 39 | ODSDA | 0.50 | NMP | $C_6H_{12}$ | A | 45 | --- | TEA | 56.0 | 0.72 | 23.2 | 20.1 |
| 40 | DSDB | 1.00 | DMF | $C_7H_{16}$ | B | 30 | 41 | DEA | 87.3 | 0.59 | 10.3 | 1.81 |
| 41 | DSDB | 0.55 | DMF | $C_7H_8$ | B | 30 | --- | TMA | 72.0 | 0.00 | 0.00 | 2.49 |

| EXP[1] | AGENT[2] | EQUIV[3] | SOLV[4] | COSOLV[5] | ESTER[6] | TIME[7] | $H_2O$[8] | AMINE[9] | MOLAR % YIELD OR % RECOVERY[10] | | | |
|---|---|---|---|---|---|---|---|---|---|---|---|---|
| | | | | | | | | | S6E[11] | MONO[12] | DI[13] | SUC[14] |
| 42 | DSDB | 0.60 | DMF | $C_7H_8$ | B | 30 | --- | TEA | 77.1 | 0.00 | 0.61 | 1.75 |
| 43 | DSDB | 0.60 | DMF | $C_7H_8$ | B | 30 | --- | DMDA | 60.0 | 0.29 | 0.82 | 3.94 |
| 44 | DSDB | 0.62 | DMF | $C_7H_8$ | B | 30 | --- | DMOA | 70.1 | 0.00 | 1.06 | 1.33 |
| 45 | DSDB | 0.62 | DMF | $C_7H_8$ | B | 30 | --- | COLL | 74.5 | 0.41 | 0.40 | 7.94 |

[1]Example number. [2]Organotin agent employed. DSDA stands for 1,3-diacetoxy-1,1,3,3-tetrabutyldistannoxane. DSDB stands for 1,3-dibenzoyloxy-1,1,3,3-tetrabutyldistannoxane. ODSDA stands for 1,3-diacetoxy-1,1,3,3-tetraoctyldistannoxane. [3]Molar equivalents of organotin agent employed basis sucrose. [4]DMF is N,N-dimethylformamide. NMP is N-methyl-2-pyrrolidone. [5]$C_7H_8$ is toluene. $C_6H_{12}$ is cyclohexane. $C_7H_{16}$ is n-heptane. [6]Sucrose-6-ester prepared. A is for acetate and B is for benzoate. [7]Time employed for reaction mixture dehydration in min. [8]Percent of total $H_2O$ present produced during reaction mixture dehydration (Karl Fischer method). [9]Tertiary amine employed. TEA is triethylamine. PYR is pyridine. DEA is diisopropylethylamine. LUT is 2,6-lutidine. TMA is trimethylamine. DMDA is N,N-dimethyldodecylamine. DMOA is N,N-dimethyloctylamine. COLL is 2,4,6-collidine. [10]Molar percent yield or percent recovery as determined by HPLC analysis of purified syrups (experiments 27 through 40) or isolated solids (experiments 41 through 45). [11]Percent yield of sucrose-6-ester. [12]Percent yield of other sucrose monoesters. [13]Percent yield of sucrose diesters. [14]Percent recovery of unreacted sucrose.

Example 27

PREPARATION OF SUCROSE-6-ACETATE USING TRIETHYLAMINE AND 0.50 EQUIVALENT DISTANNOXANE DIACETATE WITHOUT DEHYDRATION

A 1000-ml, three-neck, round-bottom flask, equipped with mechanical stirrer, thermometer, and 60-ml dropping funnel topped with an argon inlet, was charged with 68.5 g (200 mmol) of sucrose, 61.2 g (100 mmol) of DSDA•$H_2O$, and 450 ml of DMF. The slurry was heated to 75 °C (internal temperature) for 10 min, and the clear solution thus produced cooled to room temperature and treated sequentially with 50 ml of toluene and 22.3 g (220 mmol) of triethylamine. The solution was then treated dropwise over 10 min with 22.5 g (220 mmol) of acetic anhydride dissolved in 50 ml of DMF. The anhydride addition produced an about 10 °C exotherm.

The formation of S-6-A and the disappearance of sucrose were followed by silica-gel TLC. The conversion appeared to be complete after about 30 min. The reaction mixture was treated with water (50 ml), extracted with cyclohexane (2 x 500 ml) to remove DSDA•$H_2O$, and the DMF-based solution partially evaporated (rotary evaporator, mechanical-pump vacuum, 30 °C water bath) to afford a tan syrup determined by HPLC analysis to contain 49.1 g (128 mmol, 64.0% yield) of sucrose-6-acetate.

Example 28

PREPARATION OF SUCROSE-6-BENZOATE USING TRIETHYLAMINE AND 0.50 EQUIVALENT DISTANNOXANE DIBENZOATE WITHOUT DEHYDRATION

The experiment of Example 27 was repeated using 74.2 g (100 mmol) of DSDB•$H_2O$ as the organotin agent and 49.8 g (220 mmol) of benzoic anhydride dissolved 50 ml of DMF for acylation. The benzoylation required about 60 min to reach completion (TLC analysis). Work-up afforded a syrup containing 64.7 g (145 mmol, 72.5% yield) of sucrose-6-benzoate.

Example 29

PREPARATION OF SUCROSE-6-ACETATE USING TRIETHYLAMINE AND 0.50 EQUIVALENT DISTANNOXANE WITH DEHYDRATION

A 1000-ml, three-neck, round-bottom flask, equipped with mechanical stirrer, thermometer, and Dean-Stark water separator topped with a reflux condenser, was charged with 68.5 g (200 mmol) of sucrose, 61.2 g (100 mmol) of DSDA•$H_2O$, 400 ml of DMF, and 100 ml of cyclohexane. The slurry was heated to reflux (97 °C reaction temperature), and the resulting solids-free mixture refluxed for 45 min. The contents of the water separator were removed, dissolved in anhydrous isopropanol, and assayed for water by the Karl Fischer method (1.30 g, 72.2 mmol, 72.2% of the total present).

The solids-free mixture was cooled to about 20 °C, and treated in one portion with 22.3 g (220 mmol) of triethylamine. The amine addition produced a mild (about 2 °C) exotherm. Acetic anhydride (22.5 g, 220 mmol) was then added dropwise over 10 min using ice-bath cooling as necessary to keep the reaction temperature below 25 °C. The reaction appeared to be complete by TLC after stirring for about 15 min at room temperature. The mixture was worked-up as described in Example 27 to provide a syrup shown by HPLC analysis to contain 57.7 g (150 mmol, 75.1% yield) of sucrose-6-acetate.

Example 30

PREPARATION OF SUCROSE-6-ACETATE USING TRIETHYLAMINE AND 0.25 EQUIVALENT DISTANNOXANE DIACETATE WITH DEHYDRATION

The experiment of Example 29 was repeated using 30.6 g (50.0 mmol) of DSDA•$H_2O$ as the organotin agent. The dehydration temperature was 92 °C, and the water produced was 116% of the total present (60-min reflux). Acetylation and work-up afforded a syrup containing 47.6 g (124 mmol, 62.0% yield) of sucrose-6-acetate.

Example 31

PREPARATION OF SUCROSE-6-ACETATE USING PYRIDINE AND 0.50 EQUIVALENT DISTANNOXANE DIACETATE WITH DEHYDRATION

The experiment of Example 29 was repeated using 17.4 g (220 mmol) of pyridine as the tertiary amine. The dehydration temperature was 99 °C, and the water produced was 66.4% of the total present (45-min reflux). The acetylation required about 60 min to reach completion (TLC analysis). Work-up afforded a syrup containing 46.8 g (122 mmol, 60.9% yield) of sucrose-6-acetate.

Example 32

PREPARATION OF SUCROSE-6-ACETATE USING DIISOPROPYLETHYLAMINE AND 0.50 EQUIVALENTS DISTANNOXANE DIACETATE WITH DEHYDRATION

The experiment of Example 29 was repeated using 28.4 g (220 mmol) of diisopropylethylamine as the tertiary amine. The dehydration temperature was 100 °C, and the water produced was 76.5% of the total present (45-min reflux). Work-up afforded a syrup containing 55.7 g (145 mmol, 72.6% yield) of sucrose-6-acetate.

Example 33

PREPARATION OF SUCROSE-6-ACETATE USING TRIETHYLAMINE AND 0.50 EQUIVALENT DISTANNOXANE DIBENZOATE WITH DEHYDRATION

The experiment of Example 29 was repeated using 74.2 g (100 mmol) of DSDB•$H_2O$ as the organotin agent. The dehydration temperature was 102 °C, and the water produced was 60.5% of the total present (30-min reflux). The acetylation was complete after about 15 min (TLC analysis). Work-up afforded a syrup containing 58.5 g (152 mmol, 76.2% yield) of sucrose-6-acetate. No sucrose-6-benzoate could be detected in the syrup by TLC analysis.

Example 34

PREPARATION OF SUCROSE-6-ACETATE USING 2,6-LUTIDINE AND 0.50 EQUIVALENT DISTANNOXANE DIACETATE WITH DEHYDRATION

The experiment of Example 29 was repeated using 23.6 g (220 mmol) of 2,6-lutidine as the tertiary amine. The dehydration temperature was 99 °C, and the water produced was 78.2% of the total present (45-min reflux). The acetylation required about 60 min to reach completion (TLC analysis). Work-up afforded a syrup containing 56.0 g (146 mmol, 72.9% yield) of sucrose-6-acetate.

Example 35

PREPARATION OF SUCROSE-6-BENZOATE USING TRIETHYLAMINE AND 0.50 EQUIVALENT DISTANNOXANE DIACETATE WITH DEHYDRATION

The experiment of Example 29 was repeated using 49.8 g (220 mmol) of benzoic anhydride dissolved in 50 ml of DMF for acylation. The dehydration temperature was 99 °C, and the water produced as 56.8% of the total present (45-min reflux). The benzoylation required about 60 min to reach completion (TLC analysis). Work-up afforded a syrup containing 72.9 g (163 mmol, 81.6% yield) of sucrose-6-benzoate.

Example 36

PREPARATION OF SUCROSE-6-ACETATE USING TRIETHYLAMINE AND 1.00 EQUIVALENT DISTANNOXANE DIBENZOATE WITH DEHYDRATION

The experiment of Example 29 was repeated using 148 g (200 mmol) of DSDB•$H_2O$ as the organotin agent. The dehydration temperature was 99 °C, and the water produced was 39.8% of the total present (30-min reflux). Acetylation and work-up afforded a syrup containing 64.2 g (167 mmol, 83.5% yield) of sucrose-6-acetate.

Example 37

PREPARATION OF SUCROSE-6-BENZOATE USING TRIETHYLAMINE AND 1.00 EQUIVALENT DISTANNOXANE DIBENZOATE WITH DEHYDRATION

The experiment of Example 29 was repeated using 148 g (200 mmol) of DSDB•$H_2O$ as the organotin agent, 150 ml of cyclohexane as the dehydration cosolvent, and 49.8 g (220 mmol) of benzoic anhydride dissolved in 50 ml of DMF for acylation. The dehydration temperature was 95 °C, and the water produced was 45.6% of the total present (45-min reflux). Benzoylation and work-up produced a syrup containing 77.0 g (171 mmol, 86.2% yield) of sucrose-6-benzoate.

Example 38

PREPARATION OF SUCROSE-6-ACETATE USING TRIETHYLAMINE AND 0.75 EQUIVALENT DISTANNOXANE DIBENZOATE WITH DEHYDRATION

The experiment of Example 29 was repeated using 111 g (150 mmol) of DSDB•$H_2O$ as the organotin agent. The dehydration temperature was 104 °C, and the water produced was 46.8% of the total present (30-min reflux). Acetylation and work-up afforded a syrup containing 63.8 g (166 mmol, 83.0% yield) of sucrose-6-acetate.

Example 39

PREPARATION OF SUCROSE-6-ACETATE USING TRIHETHYLAMINE AND 0.50 EQUIVALENT 1,3-DIACETOXY-1,1,3,3-TETRAOCTYLDISTANNOXANE IN N-METHYL-2-PYRROLIDONE WITH DEHYDRATION

Tetraoctyldistannoxane diacetate monohydrate was prepared by dissolving 75.8 g (200 mmol) of DOTO•$\frac{1}{2}H_2O$ in 500 ml of glacial acetic acid at room temperature (about 60 min required). Rotary evaporation (water-aspirator vacuum, 40 °C bath) afforded the product as a pale-yellow viscous oil. The oil was dissolved in 500 ml of NMP, and the solution evaporated (rotary evaporator, mechanical-pump vacuum, 65 °C water bath) to remove residual acetic acid. The yield was assumed to be quantitative (86.0 g, 100 mmol).

The oil was treated with 400 ml of NMP and 150 ml of cyclohexane, and the experiment of Example 29 repeated. The dehydration was conducted for 45 min at 102 °C. Acetylation and work-up afforded a syrup containing 43.0 g (112 mmol, 56.0% yield) of sucrose-6-acetate.

Example 40

PREPARATION OF SUCROSE-6-BENZOATE USING DIISOPROPYLETHYLAMINE AND 1.00 EQUIVALENT DISTANNOXANE DIBENZOATE WITH DEHYDRATION

The experiment of Example 29 was repeated using 148 g (200 mmol) of DSDB•$H_2O$ as the organotin agent, 150 ml of n-heptane as the dehydration cosolvent, 28.4 g (220 mmol) of diisopropylethylamine as the tertiary amine, and 49.8 g (220 mmol) of benzoic anhydride dissolved in 50 ml of DMF for acylation. The dehydration temperature was 104 °C, and the water produced was 40.8% of the total present (30-min reflux). The benzoylation required about 60 min to reach completion (TLC analysis). Work-up afforded a syrup containing 77.9 g (175 mmol, 87.3% yield) of sucrose-6-benzoate.

Example 41

PREPARATION OF SOLID SUCROSE-6-BENZOATE USING TRIMETHYLAMINE AND 0.55 EQUIVALENT DISTANNOXANE DIBENZOATE WITH DEHYDRATION

A 1000-ml, three-neck, round-bottom flask, equipped with mechanical stirrer, thermometer, and vacuum distillation set-up, was charged with 50.0 g (146 mmol) of sucrose, 59.6 g (80.3 mmol) of DSDB•$H_2O$, and

250 ml of DMF. The slurry was heated to 80 °C (internal temperature) for 10 min, and the clear solution thus produced treated with toluene (100 ml) and available water removed by codistillation at about 100 mm of Hg and 90 °C internal temperature. The solution was then cooled to room temperature and treated sequentially with trimethylamine (9.49 g, 161 mmol, introduced as an anhydrous gas) and benzoic anhydride (36.4 g, 161 mmol). Ice-bath cooling was employed as necessary to maintain a reaction temperature of about 20 °C during the addition of anhydride.

After stirring at room temperature for about 2 hr, the DMF and TMA were removed by rotary evaporation (mechanical-pump vacuum, 30 °C water bath) to afford a viscous oil which was treated in the rotary evaporator flask at 50 °C with 250 ml of acetone. The clear solution thus obtained was cooled to room temperature, seeded with S-6-B, and stirred for about 60 min. The product was filtered, washed with acetone (3 x 50 ml), and vacuum dried (50 °C/0.5 mm of Hg/16 hr) to produce 48.0 g of an off-white solid determined by HPLC analysis to consist of 97.8% sucrose-6-benzoate (46.9 g, 105 mmol, 72.0% yield).

Example 42

PREPARATION OF SOLID SUCROSE-6-BENZOATE USING TRIETHYLAMINE AND 0.60 EQUIVALENT DISTANNOXANE DIBENZOATE WITH DEHYDRATION

The experiment of Example 41 was repeated using 65.0 g (87.6 mmol) of DSDB•$H_2O$ as the organotin agent and 16.3 g (161 mmol) of triethylamine as the tertiary amine. Benzoylation and work-up afforded 54.6 g of solid shown by HPLC assay to consist of 92.0% sucrose-6-benzoate (50.2 g, 113 mmol, 77.1% yield).

Example 43

PREPARATION OF SOLID SUCROSE-6-BENZOATE USING N,N-DIMETHYLDODECYLAMINE AND 0.60 EQUIVALENT DISTANNOXANE DIBENZOATE WITH DEHYDRATION

The experiment of Example 41 was repeated using 65.0 g (87.6 mmol) of DSDB•$H_2O$ as the organotin agent and 34.4 g (161 mmol) of N,N-dimethyldodecylamine as the tertiary amine. Benzoylation and work-up provided a syrup (containing S-6-B, DSDB, and DMDA) which, after treatment with acetone in the usual manner, afforded 46.9 g of solid shown by HPLC analysis to consist of 83.4% sucrose-6-benzoate (39.1 g, 87.6 mmol, 60.0% yield).

Example 44

PREPARATION OF SOLID SUCROSE-6-BENZOATE USING N,N-DIMETHYLOCTYLAMINE AND 0.62 EQUIVALENT DISTANNOXANE DIBENZOATE WITH DEHYDRATION

The experiment of Example 41 was repeated using 67.2 g (90.5 mmol) of DSDB•$H_2O$ as the organotin agent and 25.3 g (161 mmol) of N,N-dimethyloctylamine as the tertiary amine. Benzoylation and work-up provided a syrup (containing S-6-B, DSDB, and DMOA) which, after treatment with acetone in the usual manner, afforded 47.3 g of solid determined by HPLC analysis to consist of 96.6% sucrose-6-benzoate (45.7 g, 102 mmol, 70.1% yield).

Example 45

PREPARATION OF SOLID SUCROSE-6-BENZOATE USING 2,4,6-COLLIDINE AND 0.62 EQUIVALENT DISTANNOXANE DIBENZOATE WITH DEHYDRATION

The experiment of Example 41 was repeated using 67.2 g (90.5 mmol) of DSDB•$H_2O$ as the organotin agent and 19.5 g (161 mmol) of 2,4,6-collidine as the tertiary amine. Benzoylation and work-up provided a syrup (containing S-6-B, DSDB, and 2,4,6-collidine) which, after treatment with acetone in the usual manner, afforded 53.6 g of solid found by HPLC determination to consist of 90.6% sucrose-6-benzoate (48.6 g, 109 mmol, 74.5% yield).

Example 46

PREPARATION OF SOLID SUCROSE-6-BENZOATE USING DISTANNOXANE DIBENZOATE AND N,N-DIMETHYLOCTYLAMINE WITH ORGANOTIN AND AMINE RECYCLE

The three sequential preparations of solid sucrose-6-benzoate detailed in the table immediately below were conducted according to a procedure (original cycle described herein) in which sucrose (50.0 g, 146 mmol) and DSDB•$H_2O$ (65.0 g, 87.6 mmol) were dissolved in DMF (250 ml) at about 80 °C. The reaction mixture was treated with toluene (100 ml) and available water removed by codistillation at about 100 mm of Hg and 90 °C internal temperature. The solution was then cooled to room temperature and treated sequentially with 25.3 g (161 mmol) of DMOA and 36.3 g (161 mmol) of benzoic anhydride. Ice-bath cooling was employed to maintain a reaction temperature of about 20 °C during the addition of anhydride.

After stirring for about 15 min at room temperature, the DMF was removed by rotary evaporation (mechanical-pump vacuum, 30 °C water bath) to afford a viscous oil which was treated in the rotary evaporator flask with 250 ml of acetone at about 50 °C. The clear solution thus obtained was cooled to room temperature, seeded with S-6-B, and stirred for 2.5 hr. The solid product was filtered, washed with acetone (3 x 50 ml), and vacuum dried (50 °C/0.5 mm of Hg/16 hr) to produce an off-white solid (47.3 g) which was analyzed for sucrose-6-benzoate content by HPLC (96.6% pure, 45.7 g, 102 mmol, 70.1% yield).

The combined washes and crystallization mother liquor were evaporated (rotary evaporator, water-aspirator vacuum, 50 °C water bath) and partitioned between toluene (200 ml) and water (100 ml) containing sodium hydroxide (6.44 g, 161 mmol). The layers were separated and the aqueous portion (containing sodium benzoate) was discarded. The organic layer was evaporated (rotary evaporator, water-aspirator vacuum, 40 °C water bath) to afford a viscous oil which was dissolved in toluene (100 ml) and analyzed for DSDB•$H_2O$ by atomic absorption spectrophotometry (63.0 g, 84.9 mmol, 96.9% recovery) and for DMOA by gas chromatography (23.0 g, 146 mmol, 90.7% recovery). The above-described process was then repeated using the toluene solution and 50 g of sucrose dissolved in 250 ml of DMF.

| experiment | original | first recycle | second recycle |
|---|---|---|---|
| sucrose (equiv)[1] | 1.00 | 1.00 | 1.00 |
| fresh DSDB•$H_2O$ (equiv)[1,2] | 0.60 | 0.02 | 0.02 |
| recycled DSDB•$H_2O$ (equiv)[1,2] | 0.00 | 0.58 | 0.58 |
| total DSDB•$H_2O$ (equiv)[1,2] | 0.60 | 0.60 | 0.60 |
| fresh DMOA (equiv)[1,3] | 1.10 | 0.10 | 0.10 |
| recycled DMOA (equiv)[1,3] | 0.00 | 1.00 | 1.00 |
| total DMOA (equiv)[1,3] | 1.10 | 1.10 | 1.10 |
| anhydride (equiv)[1,4] | 1.10 | 1.10 | 1.10 |
| % yield (isolated)[5] | 70.1 | 71.4 | 72.9 |
| HPLC purity (%)[6] | 96.6 | 91.1 | 90.3 |

[1]Mol equiv basis sucrose. [2]DSDB•$H_2O$ is 1,3-dibenzoyloxy-1,1,3,3-tetrabutyldistannoxane monohydrate. [3]DMOA is N,N-dimethyloctylamine. [4]Anhydride is benzoic anhydride. [5]Yield of isolated solid product corrected for HPLC purity. [6]Purity of the isolated solid product as determined by HPLC.

Example 47

PREPARATION OF SOLID SUCROSE-6-BENZOATE USING DISTANNOXANE DIBENZOATE AND TRIETHYLAMINE WITH ORGANOTIN RECYCLE

The five sequential preparations of solid sucrose-6-benzoate delineated in the table immediately below were conducted according to a procedure (original cycle described herein) in which sucrose (50.0 g, 146 mmol) and DSDB•$H_2O$ (65.0 g, 87.6 mmol) were dissolved in DMF (250 ml) at about 80°C. The reaction mixture was treated with toluene (100 ml) and available water removed by codistillation at about 100 mm of Hg and 90°C internal temperature. The solution was then cooled to room temperature and treated sequentially with triethylamine (16.3 g, 161 mmol) and benzoic anhydride (36.3 g, 161 mmol). Ice-bath cooling was employed as necessary to maintain a reaction temperature of about 20°C during the addition of anhydride.

After stirring for about 15 min at room temperature, the DMF and TEA were removed by rotary evaporation (mechanicalpump vacuum, 30°C water bath) to afford a viscous oil which was treated in the rotary evaporator flask with 250 ml of acetone at about 50°C. The clear solution thus obtained was cooled to room temperature, seeded with S-6-B, and stirred for 3.0 hr. The product was filtered, washed with acetone (3 x 50 ml), and vacuum dried (50°C/0.5 mm of Hg/16 hr) to produce an off-white solid (51.8 g) which was analyzed for sucrose-6-benzoate content by HPLC (95.3% pure, 49.4 g, 111 mmol, 75.7% yield).

The combined washes and crystallization mother liquor were evaporated (rotary evaporator, water-aspirator vacuum, 30 °C water bath) and the residue partitioned between toluene (200 ml) and water (100 ml) containing sodium hydroxide (6.44 g, 161 mmol). The layers were separated and the aqueous portion (containing sodium benzoate) was discarded. The organic layer was exhaustively evaporated (rotary evaporator, water-aspirator vacuum, 40 °C water bath followed by mechanical-pump vacuum, 50 °C water bath) to afford a viscous oil which was dissolved in toluene (100 ml) and analyzed for DSDB•$H_2O$ by atomic absorption spectrophotometry (65.0 g, 87.6 mmol, 100% recovery). The above-described process was then repeated using the toluene solution and 50 g of sucrose dissolved in 250 ml of DMF.

| experiment | original | first recycle | second recycle | third recycle | fourth recycle |
|---|---|---|---|---|---|
| sucrose (equiv)[1] | 1.00 | 1.00 | 1.00 | 1.00 | 1.00 |
| fresh DSDB•$H_2O$ (equiv)[1,2] | 0.60 | 0.00 | 0.00 | 0.06 | 0.01 |
| recycled DSDB•$H_2O$ (equiv)[1,2] | 0.00 | 0.60 | 0.60 | 0.54 | 0.59 |
| total DSDB•$H_2O$ (equiv)[1,2] | 0.60 | 0.60 | 0.60 | 0.60 | 0.60 |
| TEA (equiv)[1,3] | 1.10 | 1.10 | 1.10 | 1.10 | 1.10 |
| anhydride (equiv)[1,4] | 1.10 | 1.10 | 1.10 | 1.10 | 1.10 |
| % yield (isolated)[5] | 75.7 | 71.7 | 71.3 | 67.5 | 73.1 |
| HPLC purity (%)[6] | 95.3 | 89.8 | 93.7 | 90.4 | 92.6 |

[1]Mol equiv basis sucrose. [2]DSDB•$H_2O$ is 1,3-dibenzoyloxy-1,1,3,3-tetrabutyldistannoxane monohydrate. [3]TEA is triethylamine. [4]Anhydride is benzoic anhydride. [5]Yield of isolated solid product corrected for HPLC purity. [6]Purity of the isolated solid product as determined by HPLC.

Example 48

PREPARATION OF SUCROSE-6-ACETATE SYRUP USING DISTANNOXANE DIACETATE AND TRIETHYLAMINE WITH ORGANOTIN RECYCLE

The five sequential preparations of sucrose-6-acetate syrup detailed in the table immediately below were conducted according to a procedure (original cycle described herein) in which sucrose (68.5 g, 200 mmol) and DSDA•$H_2O$ (122 g, 200 mmol) were refluxed for 60 min in a mixture of DMF (400 ml) and cyclohexane (150 ml) with codistillative water removal (93 °C dehydration temperature, 62.5% of the total water present removed). The biphasic, but solids-free, mixture was then cooled to 20 °C and sequentially treated with triethylamine (22.3 g, 220 mmol) and acetic anhydride (22.5 g, 220 mmol). Ice-bath cooling was employed as necessary to maintain a reaction temperature of about 20 °C during the anhydride addition.

After stirring for about 15 min at room temperature, the reaction mixture was treated with water (50 ml) and extracted with cyclohexane (3 x 500 ml) to remove DSDA•$H_2O$.The carbohydrate-containing solution was then evaporated (rotary evaporator, mechanical-pump vacuum, 45°C water bath) to give a tan syrup determined by HPLC analysis to contain 60.2 g (157 mmol, 78.4% yield) of sucrose-6-acetate.

The combined cyclohexane extracts were evaporated (rotary evaporator, water-aspirator vacuum, 30°C water bath) and the viscous oil thus produced dissolved in 150 ml of cyclohexane and assayed for DSDA•$H_2O$ by atomic absorption spectrophotometry (119 g, 194 mmol, 97.2% recovery). The above described process was then repeated using the cyclohexane solution and 68.5 g of sucrose dissolved in 400 ml of DMF.

| experiment | original | first recycle | second recycle | third recycle | fourth recycle |
|---|---|---|---|---|---|
| sucrose (equiv)[1] | 1.00 | 1.00 | 1.00 | 1.00 | 1.00 |
| fresh DSDA•$H_2O$ (equiv)[1,2] | 1.00 | 0.03 | 0.01 | 0.00 | 0.01 |
| recycled DSDA•$H_2O$ (equiv)[1,2] | 0.00 | 0.97 | 0.99 | 1.00 | 0.99 |
| total DSDA•$H_2O$ (equiv)[1,2] | 1.00 | 1.00 | 1.00 | 1.00 | 1.00 |
| TEA (equiv)[1,3] | 1.10 | 1.10 | 1.10 | 1.10 | 1.10 |
| anhydride (equiv)[1,4] | 1.10 | 1.10 | 1.10 | 1.10 | 1.10 |
| % yield (HPLC)[5] | 78.4 | 79.1 | 77.9 | 79.3 | 80.1 |

[1]Mol equiv basis sucrose. [2]DSDA•$H_2O$ is 1,3-diacetoxy-1,1,3,3-tetrabutyldistannoxane monohydrate. [3]TEA is triethylamine. [4]Anhydride is acetic anhydride. [5]Yield of S-6-A in the product syrup by HPLC analysis.

The process of Neiditch et al. is outlined as follows:

The process is carried out by reacting sucrose with a di(hydrocarbyl)tin oxide such as dibutyltin oxide in an inert organic vehicle. In place of the DBTO there can be used other di(hydrocarbyl)tin oxides in which the hydrocarbyl groups bonded to tin can be, individually, alkyl, cycloalkyl, aryl, or arylalkyl such as, for example, methyl, ethyl, propyl, butyl, octyl, benzyl, phenethyl, phenyl, naphthyl, cyclohexyl, and substituted phenyl. The preferred hydrocarbyl groups are alkyl having up to eight carbon atoms. In place of the tin oxide, a di(hydrocarbyl)tin dialkoxide, dihalide, diacylate, or other organic tin compound can be used as long as it generates the di(hydrocarbyloxy)distannoxane in situ.

The DHTO and sucrose may be employed in a wide range of stoichiometric ratios. However, stoichiometric ratios of about one-to-one are preferred. This is because the use of an excess of sucrose leads to contamination of the S-6-E by sucrose and undesired sucrose esters, while the use of excess DHTO causes contamination of the S-6-E product by sucrose diesters. The most preferred stoichiometric ratio uses the DHTO in a very slight (1-3%) molar excess (basis sucrose) in order to insure the near absence of sucrose in the product.

The process of Neiditch et al. is carried out in an inert organic reaction vehicle. By "inert" is meant that the reaction vehicle is free of any organic functional groups that will react with either the sucrose or the DHTO. In many cases, in order to accomplish the objectives of the invention, the inert organic reaction

vehicle will be a mixed solvent system comprising a polar aprotic solvent and a cosolvent. The polar aprotic solvent is employed for the purpose of dissolving the sucrose, and the cosolvent is employed for the purpose of codistillatively removing all water generated by the reaction of sucrose with the DHTO and also promoting the solubility of the DHTO. The polar aprotic solvents which can be employed include those described below with respect to the acylation step. DMF is the preferred polar aprotic solvent.

Cosolvents capable of codistillatively removing the water of condensation include chlorinated hydrocarbons such as chloroform, a variety of saturated and aromatic hydrocarbons such as hexane, heptane, octane, cyclohexane, benzene, and toluene, ketones such as methyl ethyl ketone and methyl isobutyl ketone, acyclic and cyclic ethers such as tetrahydrofuran, and other inert organic liquids that meet the criteria set forth herein. A very wide range of organic liquids are suitable for use as cosolvents in the invention. The primary criteria for a cosolvent are (1) that is produce a mixture with the polar aprotic solvent, the DHTO, and the sucrose, which refluxes at atmospheric pressure with an internal reaction temperature within the range of from about 75 °C to about 125 °C, (2) that it codistill the water produced by the condensation of the DHTO and sucrose, thereby facilitating removal of water during the reaction, and (3) that it promote the solubility of the DHTO in the reaction mixture (since DHTO's are usually not soluble to any significant degree in polar aprotic solvents) and thereby enhance the rate of reaction of the DHTO with sucrose.

Cosolvents which are immiscible with water and which do form a constant-composition minimum-boiling azeotrope with water are preferred, but, as has been determined by experimentation, reaction systems employing such cosolvents typically reflux at temperatures significantly higher than either the water-azeotrope boiling point or the boiling point of the pure solvent. There is also data showing that the water-cosolvent compositions of the distillates arising from these systems are not constant throughout the DHTO-sucrose condensation period.

Preferred cosolvents for reasons of chemical stability, efficiency of water removal, cost, and boiling point include cyclohexane, n-heptane, and isooctane.

The reaction between sucrose and the DHTO is carried out at a temperature within the range of from about 75 °C to about 125 °C. Below 75 °C, the reaction becomes uneconomically slow, and above 125 °C there is a tendency for the carbohydrate to decompose. The preferred reaction temperature is within the range of about 80 °C to about 100 °C, and more preferably, from about 85 °C to about 90 °C.

The product of the reaction of sucrose and DHTO is a 1,3-di-(6-O-sucrose)-1,1,3,3-tetra(hydrocarbyl)-distannoxane, which may be acylated as described below.

It is preferred to employ slightly (1-5%) more than one molar equivalent of acylating agent (basis sucrose). The selection of the particular acylating agent to be used in the acylation reaction is dictated in part by the use to which the acylated product is to be put. For example, if the acyl group is being employed as a blocking group, as it would be in the preparation of the artificial sweetener as discussed above in the Background of the Invention section of this application, an acylating agent such as benzoic or acetic anhydride would be employed because it is inexpensive, the acyl group is readily removed at an appropriate stage of the synthesis, and it is stable to reactions that the acylated compound must undergo prior to removal of the acyl group. If a S-6-E is to be the ultimate product of the synthesis, then the acylating agent used is the one that will generate the desired acyl group for the ester product.

With these principles in mind, among the acylating agents that can be used are the various anhydrides of benzoic and substituted benzoic acid (e.g., 4-nitrobenzoic acid, 3,5-dinitrobenzoic acid, and the like), alkanoic acids such as acetic acid, propionic acid, butyric acid, cyclohexanecarboxylic acid, long chain fatty acids, both saturated and unsaturated, such as stearic acid, oleic acid, linoleic acid, and the like, having up to, for example, 28 carbon atoms, unsaturated acids such as acrylic acid and methacrylic acid, substituted acids such chloroacetic acid, cyanoacetic acid, phenoxyacetic acid, and the like, and saturated and unsaturated dicarboxylic acids such as phthalic acid, maleic acid, glutaric acid, and the like.

The acylation reaction is carried out in an inert organic reaction vehicle such as DMF or other polar aprotic solvents such as DMSO, NMP, DMA, HMPA, and other polar aprotic solvents in which sucrose is soluble. DMF is the preferred polar aprotic solvent because of its low cost, its relatively low boiling point, and its suitability as a solvent for further steps in the process for producing sucralose. The acylation reaction is carried out at a temperature and for a period of time sufficient to prepare the S-6-E product.

If the anhydride is a liquid, it may be added neat to the sucrose-organotin adduct, or it may be diluted with an inert cosolvent. If the anhydride is a solid, it may be added in the solid for or added as a solution in an appropriate inert solvent. The anhydride may be added all at once, or it may be added slowly over a period of time.

Anhydride stoichiometry is an important aspect of the successful practice of this invention. The use of too little anhydride will result in a S-6-E product contaminated by residual sucrose. The use of too much

anhydride will cause sucrose diester contamination. The most preferred stoichiometric ratio uses the anhydride in a slight (5-10%) molar excess (basis sucrose) in order to insure the near absence of sucrose in the product.

Acylation temperatures from below 0 °C to about 30 °C have been employed experimentally. The upper limit of acceptable acylation temperatures is governed by the onset of thermally activated nonregioselective acylation reactions which will result in the formation of undesirable sucrose mono- and diesters. From a practical standpoint, this temperature limit is a function of the reactivity of the acid anhydride. For example, because acetic anhydride is a relatively reactive species, acylations with it are normally carried out below about 20 °C. Benzoic anhydride, on the other hand, being somewhat less reactive, allows for acylation at room temperature or slightly above.

The acylation reactions are mildly exothermic. Depending upon initial reaction temperature and rate of anhydride addition to the di(hydrocarbyl)tin-sucrose adduct, external cooling of the acylation process might be required in order that thermally activated nonregioselective acylation be minimized.

The times required for the acylations of the sucrose adducts to go to completion are dependent upon the concentration of the reactants (as the acylation is a multiple-order process), the reactivity of the acylating agent, and the temperature of the reaction mixture. Although times of from one hour to several days have been employed in the laboratory, there is no advantage to extending the reaction period longer than that time necessary for consumption of the acylating agent. This is generally complete within from about one to about five hours under typical conditions.

The products of the acylation reaction are a sucrose-6-ester and a distannoxane diester.

## Claims

1. A process for producing a sucrose-6-ester which comprises reacting sucrose with a carboxylic acid anhydride in a reaction mixture comprising a first solvent which is a polar aprotic solvent and a catalytic quantity of a 1,3-diacyloxy-1,1,3,3-tetra(hydrocarbyl)distannoxane, at a temperature within the range of from 0 °C to 60 °C, in the absence of 1,3-di(hydrocarbyloxy)-1,1,3,3-di(hydrocarbyl)distannoxane compounds, and recovering the sucrose-6-ester thereby produced.

2. The process of claim 1 wherein the 1,3-diacyloxy-1,1,3,3-tetra(hydrocarbyl)distannoxane is a 1,3-diacetoxy-1,1,3,3-tetra(hydrocarbyl)distannoxane.

3. The process of claim 1 wherein the 1,3-diacyloxy-1,1,3,3-tetra(hydrocarbyl)distannoxane is a 1,3-dibenzoyloxy-1,1,3,3-tetra(hydrocarbyl)distannoxane.

4. The process of any one of claims 1 to 3 wherein the 1,3-diacyloxy-1,1,3,3-tetra(hydrocarbyl)-distannoxane is a 1,3-diacyloxy-1,1,3,3-tetra(alkyl)distannoxane.

5. The process of claim 4 wherein the alkyl is butyl or octyl.

6. The process of any preceding claim wherein the polar aprotic solvent is *N,N*-dimethylformamide.

7. The process of claim 1 wherein the reaction mixture additionally includes a second solvent capable of removing water by codistillation, and wherein the process includes the step of codistillation to remove water from the reaction mixture.

8. The process of claim 7 wherein the second solvent is a member selected from the group consisting of hydrocarbons, chlorinated hydrocarbons, ketones and ethers.

9. The process of claim 8 wherein the second solvent is a member selected from the group consisting of toluene, cyclohexane, n-heptane, and isooctane.

10. The process of claim 1 or claim 7 wherein the 1,3-diacyloxy-1,1,3,3-tetra(hydrocarbyl)distannoxane is a member selected from the group consisting of 1,3-diacetoxy-1,1,3,3-tetrabutyldistannoxane, 1,3-dibenzoyloxy-1,1,3,3-tetrabutyldistannoxane, 1,3-diacetoxy-1,1,3,3-tetraoctyldistannoxane, and 1,3-dibenzoyloxy-1,1,3,3-tetraoctyldistannoxane wherein the first solvent is N,N-dimethylformamide, and wherein the carboxylic acid anhydride is acetic anhydride or benzoic anhydride.

11. The process of claim 10 wherein the second solvent is a member selected from the group consisting of toluene, cyclohexane, n-heptane, and isooctane.

12. The process of claim 1 or claim 11 wherein the carboxylic acid anhydride is acetic anhydride.

13. The process of claim 1 or claim 11 wherein the carboxylic acid anhydride is benzoic anhydride.

14. The process of claim 1 or claim 7 wherein said process includes the step of removing carboxylic acid from the solution of sucrose-6-ester in polar aprotic solvent, said carboxylic acid having been formed by reaction of carboxylic acid anhydride with sucrose.

15. The process of claim 12 wherein said process includes the step of removing acetic acid from the solution of sucrose-6-ester in polar aprotic solvent, said acetic acid having been formed by reaction of acetic anhydride with sucrose.

16. A process according to claim 1 which comprises:
(1) dissolving sucrose in a reaction mixture comprising a polar aprotic solvent and a 1,3-diacyloxy-1,1,3,3-tetra(hydrocarbyl)distannoxane to form a first reaction mixture;
(2) adding a carboxylic acid anhydride to first reaction mixture to form a second reaction mixture and maintaining said second reaction mixture at a temperature within the range of from 0 °C to 60 °C; and
(3) recovering the sucrose-6-ester thereby produced.

17. A process according to claim 1 which comprises:
(1) dissolving sucrose in a reaction mixture comprising a polar aprotic solvent, a second solvent capable of removing water by codistillation, and a 1,3-diacyloxy-1,1,3,3-tetra(hydrocarbyl)-distannoxane to form a first reaction mixture;
(2) subjecting said first reaction mixture to codistillation to remove water, to form thereby a second reaction mixture;
(3) adding a carboxylic acid anhydride to said second reaction mixture to form a third reaction mixture and maintaining said third reaction mixture at a temperature within the range of from 0 °C to 60 °C; and
(4) recovering the sucrose-6-ester thereby produced.

## Patentansprüche

1. Verfahren zur Herstellung eines Sucrose-6-Esters, umfassend das Umsetzen von Sucrose mit einen Carbonsäureanhydrid in einem Reaktionsgemisch, das ein erstes Solvens umfaßt, das ein polares aprotisches Solvens ist, und das eine katalytische Menge eines 1,3-Diacyloxy-1,1,3,3-tetra(hydrocarbyl) distannoxans umfaßt, bei einer Temperatur im Bereich von 0 °C bis 60 °C, in Abwesenheit von 1,3-Di-(hydrocarbyloxy)-1,1,3,3-di(hydrocarbyl)distannoxanverbindungen und Gewinnen des dabei gebildeten Sucrose-6-Esters.

2. Verfahren nach Anspruch 1, bei dein das 1,3-Diacyloxy-1,1,3,3-tetra(hydrocarbyl)distannoxan ein 1,3-Diacetoxy-1,1,3,3-tetra(hydrocarbyl)distannoxan ist.

3. Verfahren nach Anspruch 1, bei dem das 1,3-Diacyloxy-1,1,3,3-tetra(hydrocarbyl)distannoxan ein 1,3-Dibenzoyloxy-1,1,3,3-tetra(hydrocarbyl)distannoxan ist.

4. Verfahren nach einem der Ansprüche 1 bis 3, bei dem das 1,3-Diacyloxy-1,1,3,3-tetra(hydrocarbyl)-distannoxan ein 1,3-Diacyloxy-1,1,3,3-tetra(alkyl)distannoxan ist.

5. Verfahren nach Anspruch 4, bei dem das Alkyl Butyl oder Octyl ist.

6. Verfahren nach einem der vorhergehenden Ansprüche, bei dem das polare aprotische Solvens N,N-Dimethylformamid ist.

7. Verfahren nach Anspruch 1, bei dem das Reaktionsgemisch zusätzlich ein zweites Solvens enthält, das in der Lage ist. Wasser durch Codestillation zu entfernen und bei dem das Verfahren den Codestillationsschritt umfaßt, um Wasser aus dem Reaktionsgemisch zu entfernen.

8. Verfahren mach Anspruch 7, bei dem das zweite Solvens ausgewählt ist aus der Gruppe bestehend aus Kohlenwasserstoffen, chlorierten Kohlenwasserstoffen, Ketonen und Ethern.

9. Verfahren nach Anspruch 8, bei dem das zweite Solvens ausgewählt ist aus der Gruppe bestehend aus Toluol, Cyclohexan, n-Heptan und Isooctan.

10. Verfahren nach Anspruch 1 oder Anspruch 7, bei dem das 1,3-Diacyloxy-1,1,3,3-tetra(hydrocarbyl) distannoxan ausgewählt ist aus der Gruppe bestehend aus 1,3-Diacetoxy-1,1,3,3-tetrabutyldistannoxan, 1,3-Dibenzoyloxy-1,1,3,3-tetrabutyldistannoxan, 1,3-Diacetoxy-1,1,3,3-tetraoctyldistannoxan und 1,3-Dibenzoyloxy-1,1,3,3-tetraoctyldistannoxan, wobei das erste Solvens N,N-Dimethylformamid ist und wobei das Carbonsäureanhydrid Acetanhydrid oder Benzoesäureanhydrid ist.

11. Verfahren nach Anspruch 10, bei dem das zweite Solvens ausgewählt ist aus der Gruppe bestehend aus Toluol, Cyclohexan, n-Heptan und Isooctan.

12. Verfahren nach Anspruch 1 oder Anspruch 11, bei dem das Carbonsäureanhydrid Acetanhydrid ist.

13. Verfahren nach Anspruch 1 oder Anspruch 11, bei dem das Carbonsäureanhydrid Benzoesäureanhydrid ist.

14. Verfahren nach Anspruch 1 oder Anspruch 7, bei dem das Verfahren den Schritt umfaßt, daß Carbonsäure aus der Sucrose-6-Ester-Lösung in polarem aprotischen Solvens entfernt wird, wobei die Carbonsäure durch Reaktion von Carbonsäureanhydrid mit Sucrose gebildet worden ist.

15. Verfahren nach Anspruch 12, wobei das Verfahren den Schritt umfaßt, daß Essigsäure aus der Sucrose-6-Ester-Lösung in polarem aprotischen Solvens entfernt wird, wobei die Essigsäure durch Reaktion von Acetanhydrid mit Sucrose gebildet worden ist.

16. Verfahren nach Anspruch 1, umfassend:
(1) das Auflösen von Sucrose in einem Reaktionssgemisch, das ein polares aprotisches Solvens und ein 1,3-Diacyloxy-1,1,3,3-tetra(hydrocarbyl)distannoxan umfaßt, wobei ein erstes Reaktionsgemisch gebildet wird;
(2) Zugeben eines Carbonsäureanhydrids zu dem ersten Reaktionsgemisch, um ein zweites Reaktionsgemisch zu bilden und Halten des zweiten Reaktionsgemisches bei einer Temperatur im Bereich von 0 °C bis 60 °C: und
(3) Gewinnen des dabei gebildeten Sucrose-6-Esters.

17. Verfahren nach Anspruch 1, umfassend:
(1) Auflösen von Sucrose in einem Reaktionsgemisch, das ein polares aprotisches Solvens, ein zweites Solvens, das in der Lage ist, Wasser durch Codestillation zu entfernen, und ein 1,3-Diacyloxy-1,1,3,3-tetra(hydrocarbyl)distannoxan umfaßt, wobei ein erstes Reaktionsgemisch gebildet wird;
(2) Codestillieren des ersten Reaktionsgemischs, am Wasser zu entfernen, wobei ein zweites Reaktionsgemisch gebildet wird;
(3) Zugeben eines Carbonsäureanhydrids zu dem zweiten Reaktionsgemisch, wobei ein drittes Reaktionsgemisch gebildet wird und Halten des dritten Reaktionsgemisches bei einer Temperatur im Bereich von 0 °C bis 60 °C; und
(4) Gewinnen des dabei gebildeten Sucrose-6-Esters.

**Revendications**

1. Procédé pour préparer un saccharose-6-ester, qui comprend la réaction de saccharose avec un anhydride d'acide carboxylique dans un mélange réactionnel comprenant un premier solvant qui est un solvant aprotique polaire et une quantité catalytique d'un 1,3-diacyloxy-1,1,3,3-tétra(hydrocarbyl)-

distannoxane, à une température dans la gamme de 0 °C à 60 °C, en l'absence de composés 1,3-di-(hydrocarbyloxy)-1,1,3,3-di(hydrocarbyl)distannoxanes,et la récupération du sacharrose-6-ester ainsi produit.

**2.** Procédé selon la revendication 1, dans lequel le 1,3-diacyloxy-1,1,3,3-tétra(hydrocarbyl)distannoxane est un 1,3-diacétoxy-1,1,3,3-tétra(hydrocarbyl)distannoxane.

**3.** Procédé selon la revendication 1, dans lequel le 1,3-diacyloxy-1,1,3,3-tétra(hydrocarbyl)distannoxane est un 1,3-dibenzoyloxy-1,1,3,3-tétra(hydrocarbyl)distannoxane.

**4.** Procédé selon l'une quelconque des revendications 1 à 3, dans lequel le 1,3-diacyloxy-1,1,3,3-tétra-(hydrocarbyl)distannoxane est un 1,3-diacyloxy-1,1,3,3-tétra(alkyl)distannoxane.

**5.** Procédé selon la revendication 4, dans lequel le radical alkyle est le radical butyle ou octyle.

**6.** Procédé selon l'une quelconque des revendications précédentes, dans lequel le solvant aprotique polaire est le N,N-diméthylformamide.

**7.** Procédé selon la revendication 1, dans lequel le mélange réactionnel inclut en plus un second solvant capable de séparer l'eau par codistillation, et où le procédé inclut l'étape de codistillation pour séparer l'eau du mélange réactionnel.

**8.** Procédé selon la revendication 7, dans lequel le second solvant est un élément choisi dans le groupe constitué des hydrocarbures, des hydrocarbures chlorés, des cétones et des éthers.

**9.** Procédé selon la revendication 8, dans lequel le second solvant est un élément choisi dans le groupe constitué du toluène, du cyclohexane, du n-heptane et de l'isooctane.

**10.** Procédé selon la revendication 1 ou la revendication 7, dans lequel le 1,3-diacyloxy-1,1,3,3-tétra-(hydrocarbyl)distannoxane est un élément choisi dans le groupe constitué du 1,3-diacétoxy-1,1,3,3-tétrabutyldistannoxane, du 1,3-dibenzoyloxy-1,1,3,3-tétrabutyldistannoxane, du 1,3-diacétoxy-1,1,3,3-tétraoctyldistannoxane et du 1,3-dibenzoyloxy-1,1,3,3-tétraoctyldistannoxane, dans lequel le premier solvant est le N,N-diméthylformamide et dans lequel l'anhydride d'acide carboxylique est l'anhydride acétique ou l'anhydride benzoïque.

**11.** Procédé selon la revendication 10, dans lequel le second solvant est un élément choisi dans le groupe constitué du toluène, du cyclohexane, du n-heptane et de l'isooctane.

**12.** Procédé selon la revendication 1 ou la revendication 11, dans lequel l'anhydride d'acide carboxylique est l'anhydride acétique.

**13.** Procédé selon la revendication 1 ou la revendication 11, dans lequel l'anhydride d'acide carboxylique est l'anhydride benzoïque.

**14.** Procédé selon la revendication 1 ou la revendication 7, dans lequel ledit procédé inclut l'étape d'élimination de l'acide carboxcylique de la solution de saccharose-6-ester dans un solvant aprotique polaire, ledit acide carboxylique ayant été formé par réaction de l'anhydride d'acide carboxylique avec du saccharose.

**15.** Procédé selon la revendication 12, dans lequel ledit procédé inclut l'étape d'élimination de l'acide acétique de la solution de saccharose-6-ester dans un solvant aprotique polaire, ledit acide acétique ayant été formé par réaction de l'anhydride acétique avec du sacharrose.

**16.** Procédé selon la revendication 1, qui comprend :

(1) la dissolution de saccharose dans un mélange réactionnel comprenant un solvant aprotique polaire et un 1,3-diacyloxy-1,1,3,3-tétra(hydrocarbyl)distannoxane pour former un premier mélange réactionnel ;

(2) l'addition d'un anhydride d'acide carboxylique au premier mélange réactionnel pour former un second mélange réactionnel, et le maintien dudit second mélange réactionnel à une température dans la gamme de 0 °C à 60 °C ; et
(3) la récupération du saccharose-6-ester ainsi produit.

**17.** Procédé selon la revendication 1, qui comprend :
(1) la dissolution de saccharose dans un mélange réactionnel comprenant un solvant aprotique polaire, un second solvant capable de séparer l'eau par codistillation, et un 1,3-diacyloxy-1,1,3,3-tétra(hydrocarbyl)distannoxanepour former un premier mélange réactionnel ;
(2) la soumission dudit premier mélange réactionnel à une codistillation pour séparer l'eau et former ainsi un deuxième mélange réactionnel ;
(3) l'addition d'un anhydride d'acide carboxylique audit deuxième mélange réactionnel pour former un troisième mélange réactionnel, et le maintien dudit troisième mélange réactionnel à une température dans la gamme de 0 °C à 60 °C ; et
(4) la récupération du saccharose-6-ester ainsi produit.

FIGURE 1

SCHEMATIC OF AMINE-ACCELERATED CONTINUOUS PROCESS USING A NONVOLATILE AMINE WITH ISOLATION OF SUCROSE-6-ESTER*

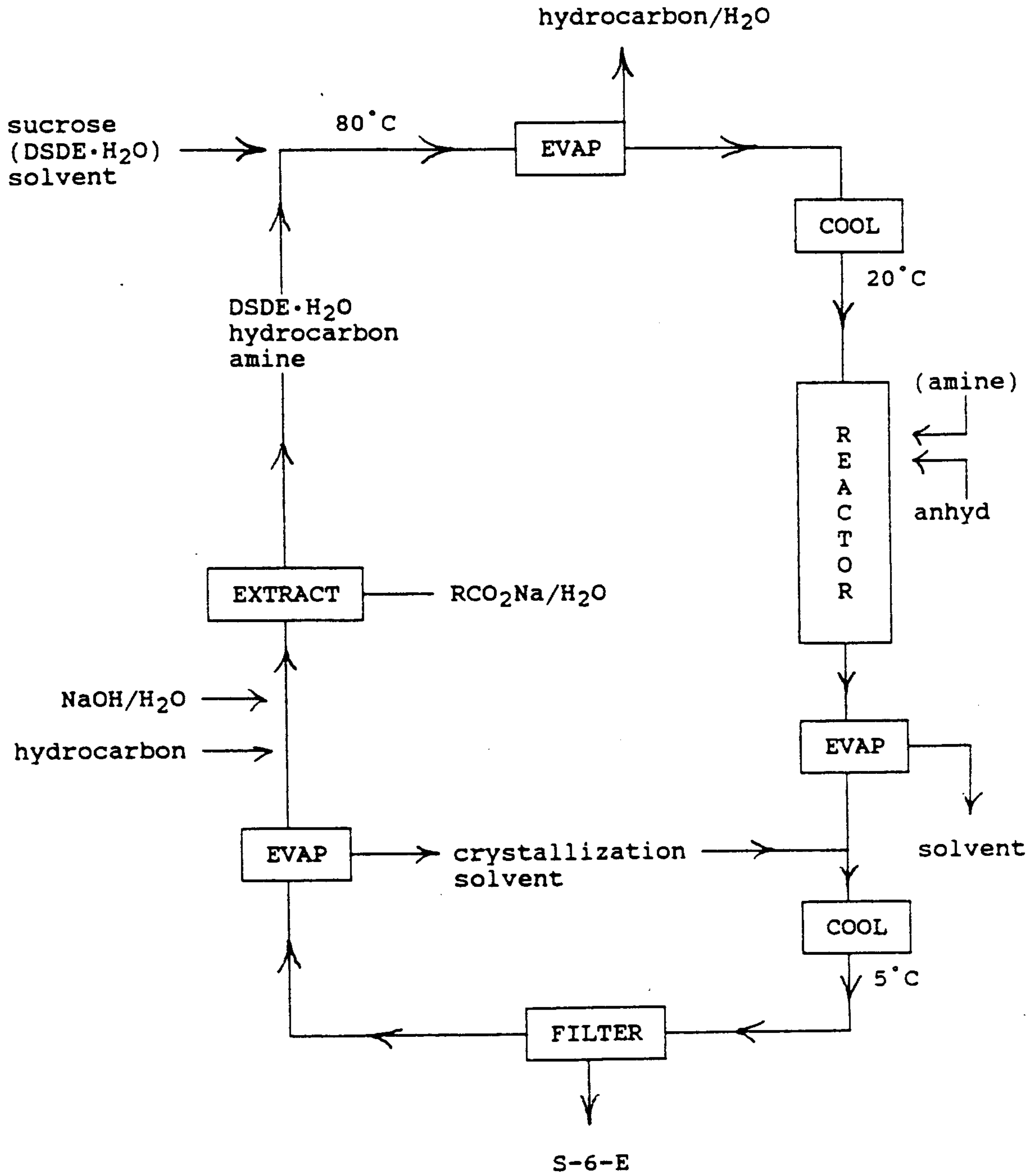


* compounds in parentheses are for make-up of losses

FIGURE 2

SCHEMATIC OF AMINE-ACCELERATED CONTINUOUS PROCESS USING A VOLATILE AMINE WITH ISOLATION OF SOLID SUCROSE-6-ESTER*

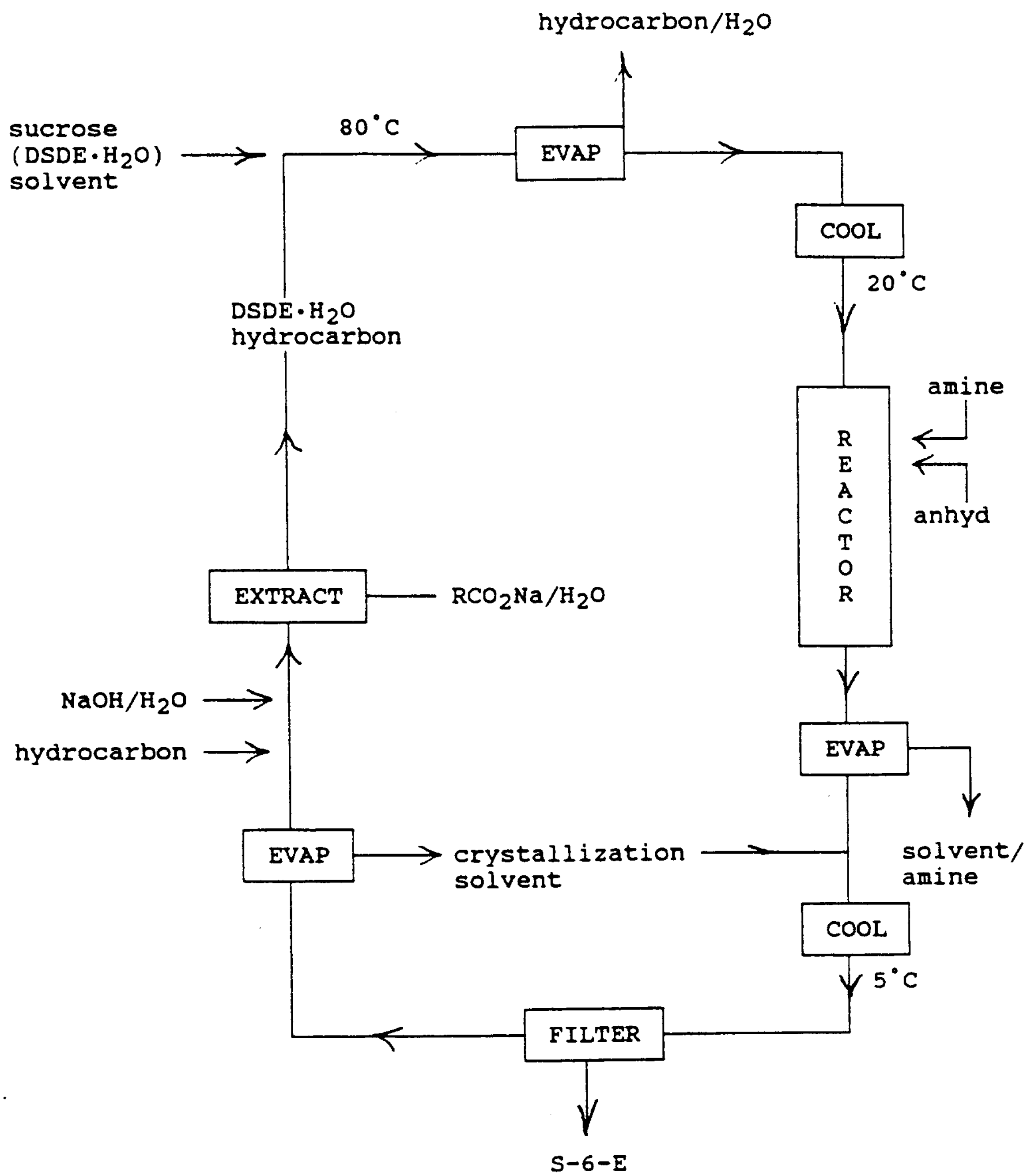


* compounds in parentheses are for make-up of losses

FIGURE 3

SCHEMATIC OF AMINE-ACCELERATED CONTINUOUS PROCESS USING A VOLATILE AMINE AND A VOLATILE CARBOXYLIC ACID BYPRODUCT WITH ISOLATION OF A SUCROSE-6-ESTER SYRUP*

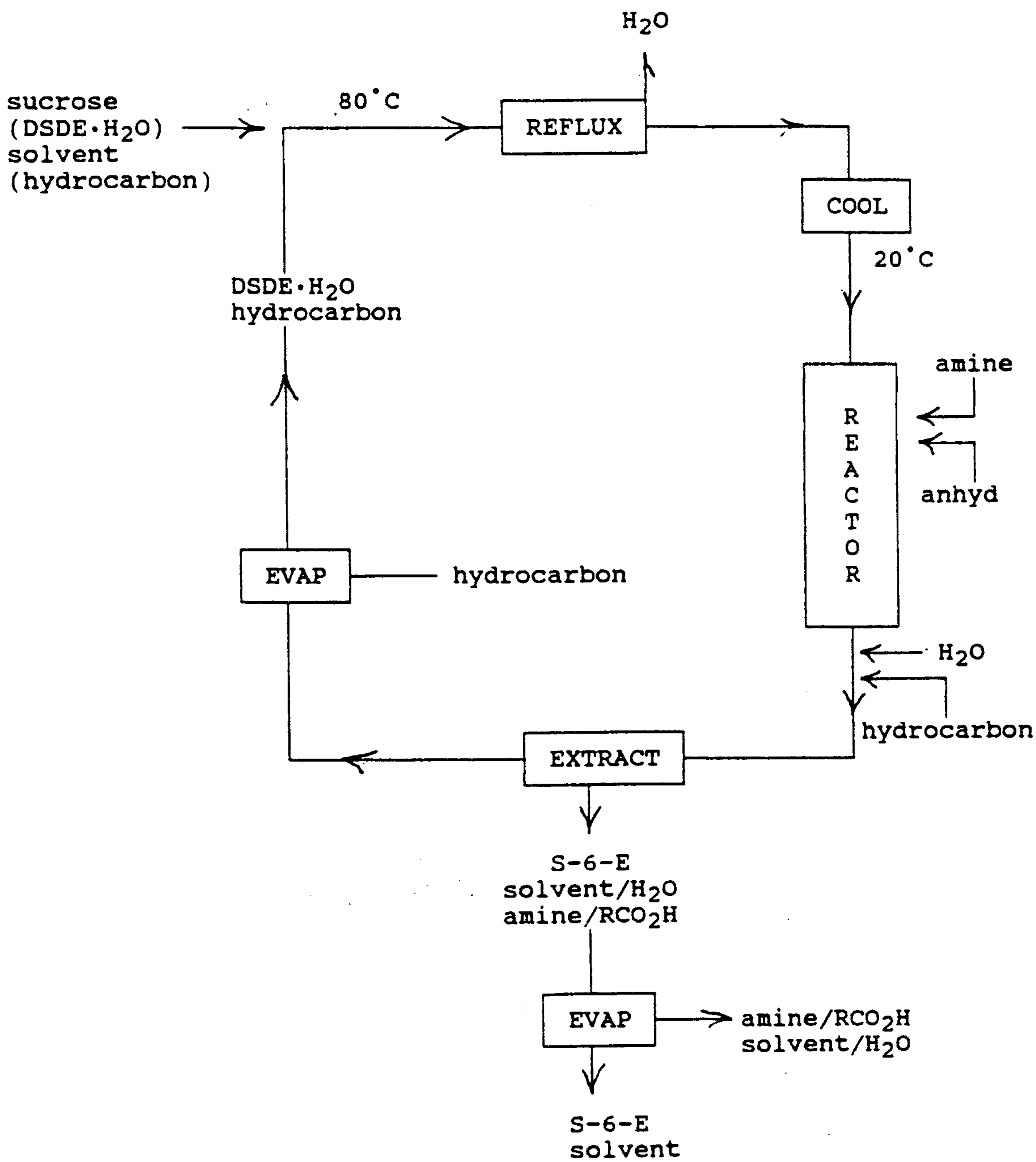


* compounds in parentheses are for make-up of losses